# EUROPEAN PATENT SPECIFICATION

(11) **EP 4 168 379 B1**
(45) Date of publication and mention of the grant of the patent: **11.02.2026**
(21) Application number: 21733448.1
(22) Date of filing: 18.06.2021
(51) Int. Cl.: H10K 85/60, C07C 255/35, C07C 255/37, C07C 255/51, C07D 213/61, C07D 213/84, C07D 239/26, C07D 251/24, H10K 50/17, H10K 101/30

(54) **ORGANIC COMPOUND OF FORMULA (I) FOR USE IN ORGANIC ELECTRONIC DEVICES, AN ORGANIC ELECTRONIC DEVICE COMPRISING A COMPOUND OF FORMULA (I) AND A DISPLAY DEVICE COMPRISING THE ORGANIC ELECTRONIC DEVICE**
ORGANISCHE VERBINDUNG VON FORMEL (I) ZUR VERWENDUNG IN ORGANISCHEN ELEKTRONISCHEN VORRICHTUNGEN, ORGANISCHE ELEKTRONISCHE VORRICHTUNG MIT EINER VERBINDUNG VON FORMEL (I) UND ANZEIGEVORRICHTUNG MIT DER ORGANISCHEN ELEKTRONISCHEN VORRICHTUNG
COMPOSÉ ORGANIQUE DE FORMULE (I) POUR UNE UTILISATION DANS DES DISPOSITIFS ÉLECTRONIQUES ORGANIQUES, DISPOSITIF ÉLECTRONIQUE ORGANIQUE COMPRENANT UN COMPOSÉ DE FORMULE (I) ET DISPOSITIF D'AFFICHAGE COMPRENANT LE DISPOSITIF ÉLECTRONIQUE ORGANIQUE

(30) Priority: 22.06.2020 EP 20181386; 22.06.2020 EP 20181398; 22.06.2020 EP 20181408; 22.10.2020 EP 20203457; 22.10.2020 EP 20203463; 22.10.2020 EP 20203460; 22.10.2020 EP 20203458; 22.10.2020 EP 20203447; 14.06.2021 WO PCT/EP2021/065949
(43) Date of publication of application: 26.04.2023
(73) Proprietor: Novaled GmbH, 01099 Dresden (DE)
(72) Inventor: NÜLLEN, Max Peter, 01099 Dresden (DE); SCHULZE, Benjamin, 01099 Dresden (DE); WUDARCZYK, Jakob Jacek, 01099 Dresden (DE)
(74) Representative: Michalski Hüttermann & Partner Patentanwälte mbB
(86) International application number: PCT/EP2021/066597
(87) International publication number: WO 2021/259782

(56) References cited:
- EP-A1- 1 988 587
- WO-A1-2019/168368
- ENOMOTO TETSUYA ET AL: "Synthesis, Structure, and Properties of Hexaaryl[3]radialenes", BULLETIN OF THE CHEMICAL SOCIETY OF JAPAN, vol. 73, no. 9, 1 September 2000 (2000-09-01), JP, pages 2109 - 2114, XP055822263, ISSN: 0009-2673, Retrieved from the Internet <URL:http://dx.doi.org/10.1246/bcsj.73.2109> DOI: 10.1246/bcsj.73.2109

## Description

### Technical Field

The present invention relates to an organic compound of formula (I) for use in organic electronic devices, an organic electronic device comprising a compound of formula (I) and a display device comprising the organic electronic device

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the semiconductor layer, and among them, may be affected by characteristics of compounds of formula (I) metal complexes which are also contained in the semiconductor layer.

There remains a need to improve performance of organic semiconductor materials, semiconductor layers, as well as organic electronic devices thereof, in particular to achieve improved operating voltage stability over time through improving the characteristics of the compounds comprised therein.

Compounds for organic optoelectronic devices and devices comprising these compounds are inter alia disclosed in the WO2019/168368 A1, EP 1 988 587 A1 and ENOMOTO TETSUYA ET AL: "Synthesis, Structure, and Properties of Hexaaryl[3]radialenes",BULLETIN OF THE CHEMICAL SOCIETY OF JAPAN,vol. 73, no. 9 1 September 2000 (2000-09-01), pages 2109-2114. DISCLOSURE

An aspect of the present invention provides an organic compound for use in organic electronic devices of formula (I): 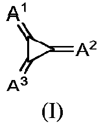 whereby A¹ is selected from formula (II) 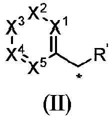
X¹ is selected from CR¹;
X² is selected from CR² or N;
X³ is selected from CR³ or N;
X⁴ is selected from CR⁴ or N;
X⁵ is selected from CR⁵ or N;
R¹ is selected from D or H, preferably H.
R², R³, R⁴ and R⁵ (if present) are independently selected from CN, partially fluorinated or perfluorinated C₁ to C₈ alkyl, Cl, F, D or H, whereby when any of R², R³, R⁴ and R⁵ is present, then the corresponding X², X³, X⁴ and X⁵ is not N;
with the proviso that either
   - at least one R² to R⁵ is selected from selected from CN, partially fluorinated or perfluorinated C₁ to C₈ alkyl, Cl, F; or
at least one X² to X⁵ is N;
A² and A³ are independently selected from formula (III) 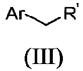
wherein Ar is independently selected from substituted or unsubstituted C₆ to C₁₈ aryl and substituted or unsubstituted C₂ to C₁₈ heteroaryl, wherein the substituents on Ar are independently selected from CN, partially or perfluorinated C₁ to C₆ alkyl, Cl, F, D; and
R' is selected from Ar, substituted or unsubstituted C₆ to C₁₈ aryl or C₃ to C₁₈ heteroaryl, partially flurorinated or perfluorinated C₁ to C₈ alkyl, F or CN,
wherein the asterix "*" denotes the binding position,
wherein the LUMO level of compound of formula (I) when calculated with the program package TURBPOMOLE V6.5 (TURBOMOLE GmbH, Litzenhardtstrasse 19, 76135 Karlsruhe, Germany) by applying the hybrid functional B3LYP with a 6-31 G* basis set in the gas phase is selected in the range of ≤ -4.7 eV and ≥ -5.25 eV;
wherein the compounds shown in claim 1 are excluded.

It should be noted that throughout the application and the claims any Aⁿ, Bⁿ, Rⁿ etc. always refer to the same moieties, unless otherwise noted.

In the present specification, when a definition is not otherwise provided, "partially fluorinated" refers to a C₁ to C₈ alkyl group in which only part of the hydrogen atoms are replaced by fluorine atoms.

In the present specification, when a definition is not otherwise provided, "perfluorinated" refers to a C₁ to C₈ alkyl group in which all hydrogen atoms are replaced by fluorine atoms.

In the present specification, when a definition is not otherwise provided, "substituted" refers to one substituted with a deuterium, C₁ to C₁₂ alkyl and C₁ to C₁₂ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a C₁ to C₁₂ alkyl group. More specifically, the alkyl group may be a C₁ to C₁₀ alkyl group or a C₁ to C₆ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an isopropyl group, a butyl group, an iso-butyl group, a sec-butyl group, a tert-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common sp²-hybridized carbon atoms

In the present specification, the single bond refers to a direct bond.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms "anode", "anode layer" and "anode electrode" are used synonymously.

The terms "cathode", "cathode layer" and "cathode electrode" are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

### Advantageous Effects

Surprisingly, it was found that the organic compound of the present invention solves the problem underlying the present invention by enabling devices in various aspects superior over the organic electroluminescent devices known in the art, in particular with respect to operating voltage over lifetime.

According to one embodiment of the present invention, the compound is selected of the formula (IV) 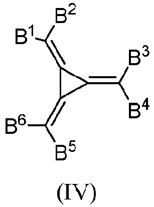 whereby B¹ is selected from formula (V) 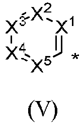 B³ and B⁵ are Ar and B², B⁴ and B⁶ are R³; wherein the asterix "*" denotes the binding position. According to one embodiment of the present invention, either R¹ and/or R⁵ are independently selected from CN, perfluorinated C₁ to C₈ alkyl, or at least one Rⁿ and Rⁿ⁺¹ (with n= 1 to 4) are independently selected from CN, perfluorinated C₁ to C₈ alkyl.
According to one embodiment of the present invention, either R¹ and/or R⁵ are independently selected from CN or CF₃, or at least one Rⁿ and Rⁿ⁺¹ (with n= 1 to 4) are independently selected from CN or CF₃.

According to one embodiment of the present invention, A¹ and A³ are not identical.

Alternatively, according to one embodiment of the present invention, at least one from A² and A³ is identical to A¹

According to one embodiment of the present invention, A¹ and A² may be selected the same or differently and A¹ and A³ are not identical.

According to one embodiment of the present invention, A¹ and A² are identical and A¹ and A³ are not identical.

According to one embodiment of the present invention, A¹, A² and A³ are identical.

According to one embodiment of the present invention, A¹, A² and A³ are identical and not selected from any of the following moieties:

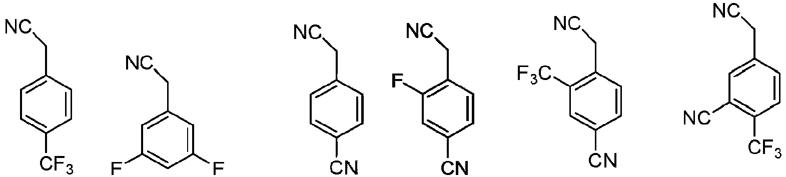

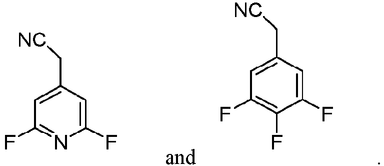

According to one embodiment of the present invention, X², X³ and/or X⁴ are selected from CN or N; preferably X², X³ and/or X⁴ are selected from CN or N and at least one remaining Rⁿ, wherein n is 2 to 5, is selected from Cl or F.

According to one embodiment of the present invention, X², X³ and/or X⁴ are selected from perfluorinated C₁ to C₈ alkyl and at least one remaining Rⁿ, wherein n is an integer from 2 to 5, is selected from Cl or F; preferably at least two remaining Rⁿ, wherein n is an integer from 2 to 5, are selected from Cl or F.

According to one embodiment of the present invention, X³ is selected from N or CR³, wherein R³ is selected from CN, partially flurorinated or perfluorinated C₁ to C₈ alkyl.

According to one embodiment of the present invention, R² and/or R³ are selected from CN, or partially flurorinated or perfluorinated C₁ to C₈ alkyl.

According to one embodiment of the present invention, one of R² and R³ is selected from CN, partially flurorinated or perfluorinated C₁ to C₈ alkyl and at least one out of R² to R⁵ is independently selected from CN, partially flurorinated or perfluorinated C₁ to C₈ alkyl, Cl, F.

According to one embodiment of the present invention, R⁵ is D or H, preferably H.

According to one embodiment of the present invention, at least two out of R² to R⁵ are independently selected from Cl, F.

According to one embodiment of the present invention, at least three out of R² to R⁵ are independently selected from CN, partially flurorinated or perfluorinated C₁ to C₈ alkyl, Cl, F.

According to one embodiment of the present invention, three R² to R⁵ are independently selected from CN, partially flurorinated or perfluorinated C₁ to C₈ alkyl, Cl, F

According to one embodiment of the present invention one of X² to X⁵ is N and one of R² to R⁵ is independently selected from CN, partially flurorinated or perfluorinated C₁ to C₈ alkyl, Cl, F.

According to one embodiment of the present invention, one of X² and X⁵ is N and one of R² to R⁵ is independently selected from CN, partially flurorinated or perfluorinated C₁ to C₈ alkyl, Cl, F.

According to one embodiment of the present invention, A¹ and A³ are not identical, and in A³ Ar is selected from substituted C₆ to C₁₂ aryl or substituted C₂ to C₁₂ heteroaryl, wherein at least one substituent on Ar is independently selected from CN or perfluorinated C₁ to C₆ alkyl, and at least two substituents are selected Cl, F; preferably Ar is selected from substituted phenyl or from group consisting of pyridyl or pyrimidyl, wherein at least one substituent on Ar is independently selected from CN or perfluorinated C₁ to C₆ alkyl, and at least two substituents are selected from Cl, F.

According to one embodiment of the present invention, A¹ and A³ are not identical, and in A³ Ar is selected from substituted C₆ to C₁₂ aryl or substituted C₂ to C₁₂ heteroaryl, wherein the substituents on Ar are independently selected from CN, perfluorinated C₁ to C₆ alkyl, Cl, F; preferably Ar is selected from substituted phenyl or from group consisting of pyridyl or pyrimidyl, wherein the substituents on Ar are independently selected from CN, perfluorinated C₁ to C₆ alkyl, Cl, F.

According to one embodiment of the present invention, A¹ and A² are not identical, whereas A² and A³ are identical and in A² and A³ Ar is selected from substituted C₆ to C₁₂ aryl or substituted C₂ to C₁₂ heteroaryl, wherein the substituents on Ar are independently selected from CN, perfluorinated C₁ to C₆ alkyl, Cl, F; preferably Ar is selected from substituted phenyl or from group consisting of pyridyl or pyrimidyl, wherein the substituents on Ar are independently selected from CN, perfluorinated C₁ to C₆ alkyl, Cl, F

According to one embodiment of the present invention, A¹ and A² are identical and A¹ and A³ are not identical; and in A³ Ar is selected from substituted C₆ to C₁₂ aryl or substituted C₂ to C₁₂ heteroaryl, wherein the substituents on Ar are independently selected from CN, perfluorinated C₁ to C₆ alkyl, Cl, F; preferably Ar is selected from substituted phenyl or from group consisting of pyridyl or pyrimidyl, wherein the substituents on Ar are independently selected from CN, perfluorinated C₁ to C₆ alkyl, Cl, F.

According to one embodiment of the present invention R' is selected CN.

According to one embodiment of the present invention, formula (II) is selected from the group comprising the following moieties:

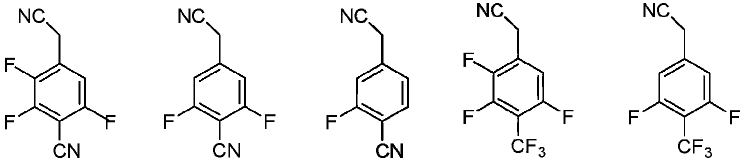

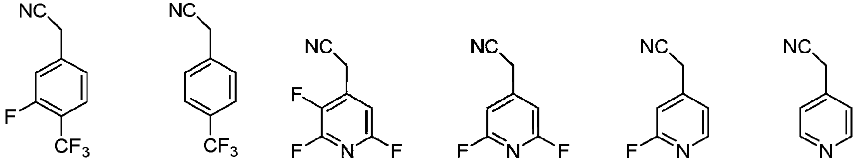

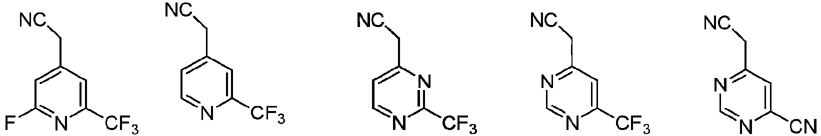

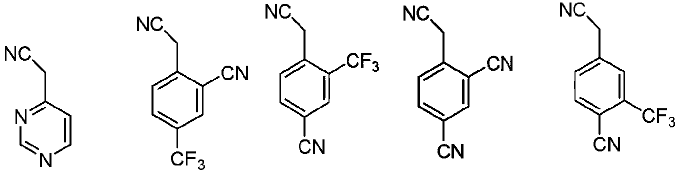

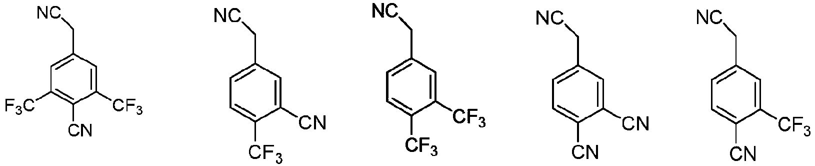

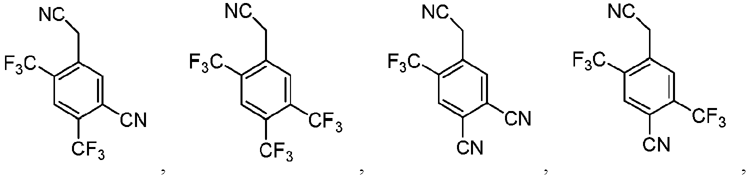

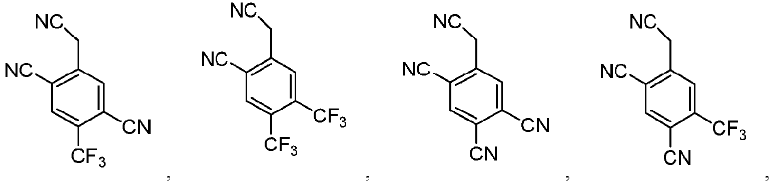

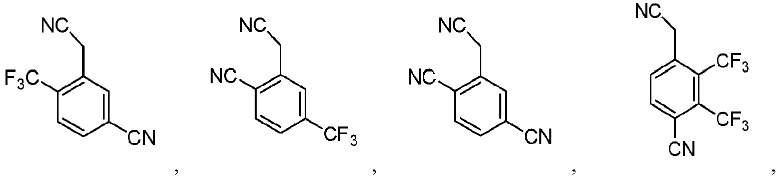

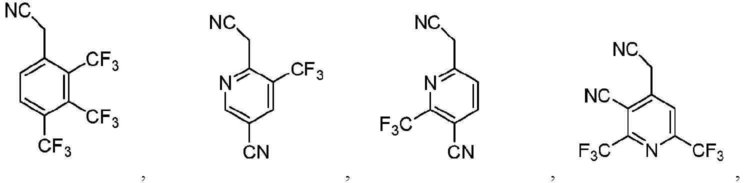

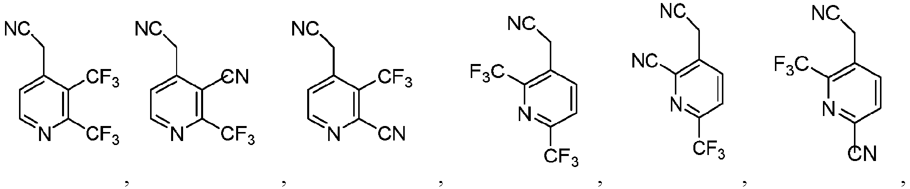

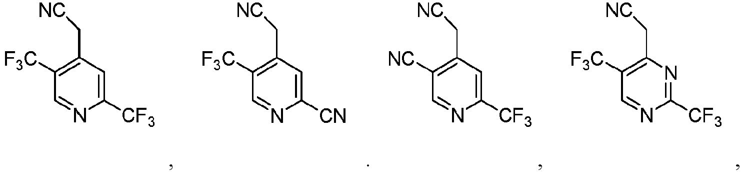

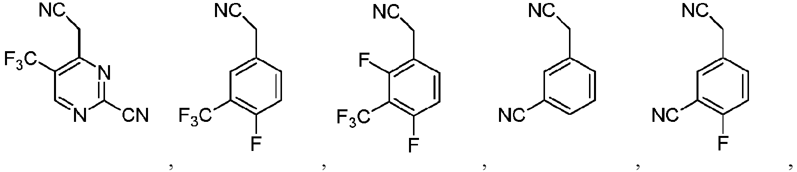

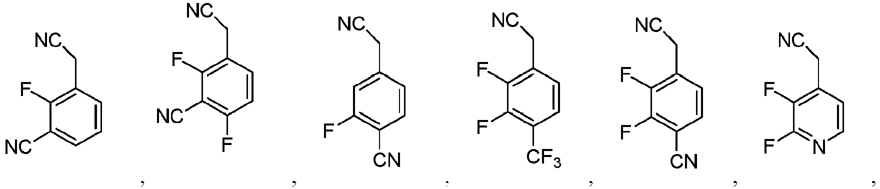

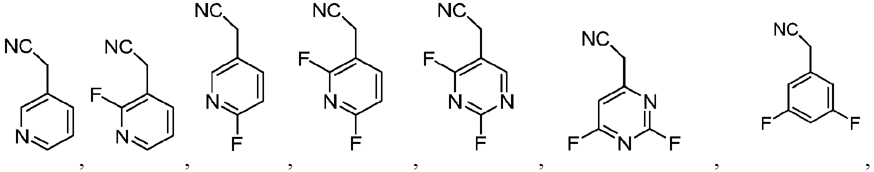

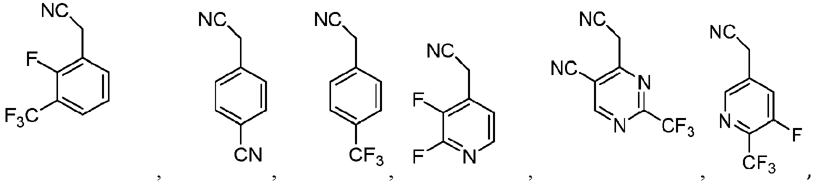

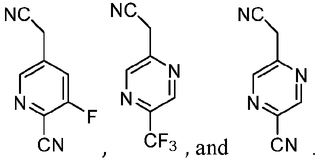

According to one embodiment of the present invention, formula (II) does not include the following moieties:

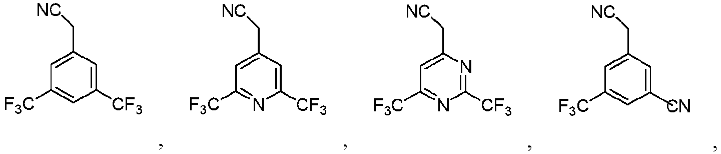

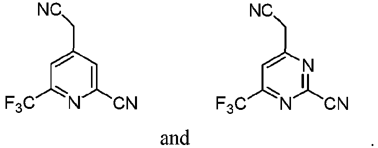

According to one embodiment of the present invention, formula (III) is selected from the group comprising the following moieties: 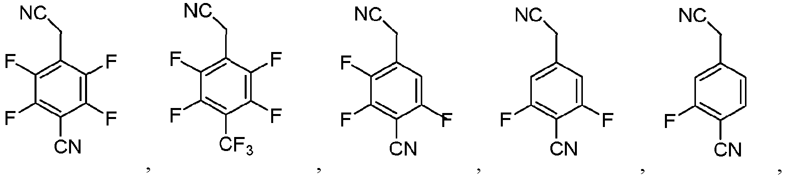 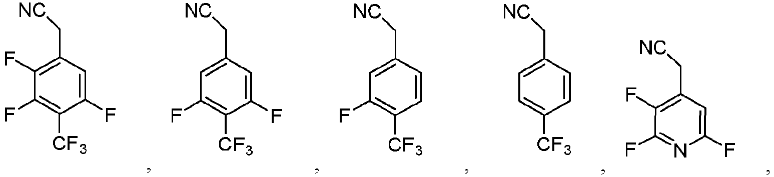 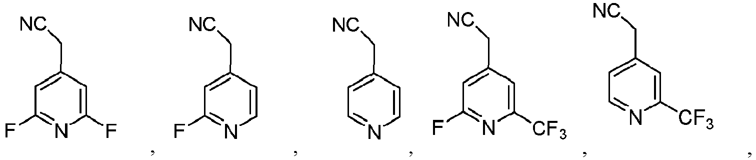 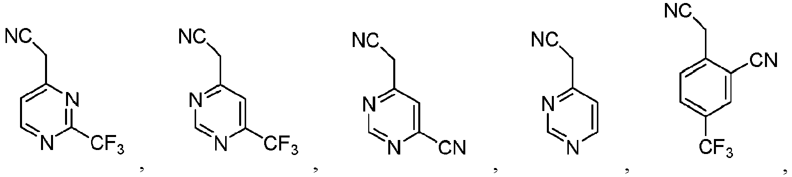 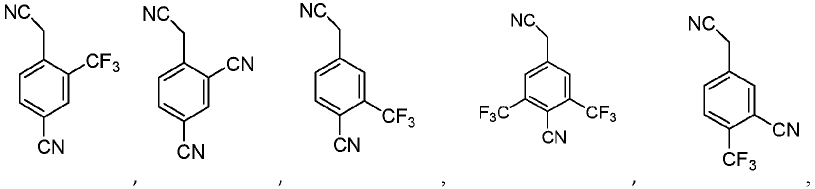 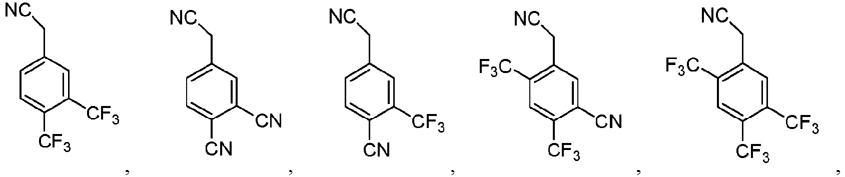 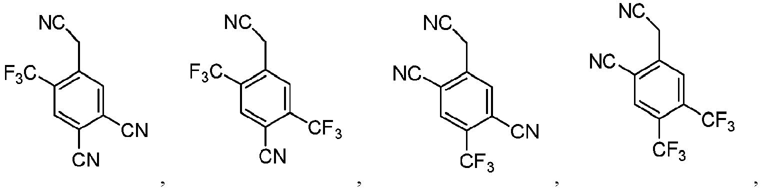 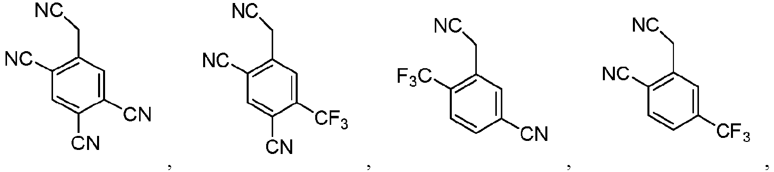 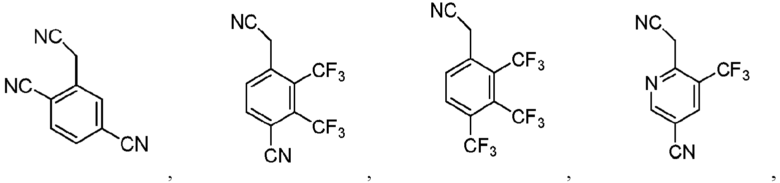 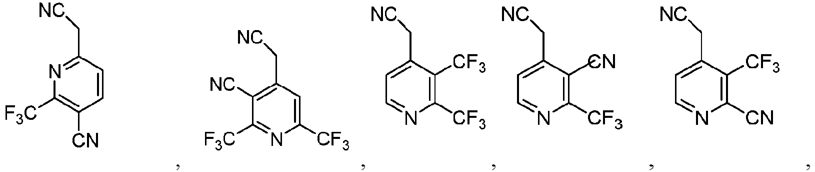 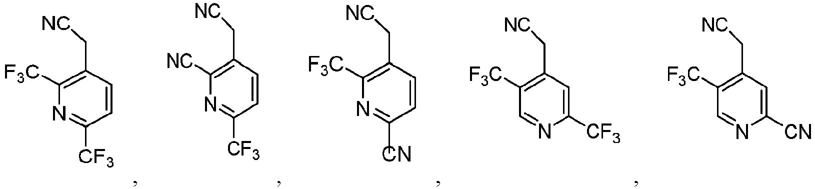 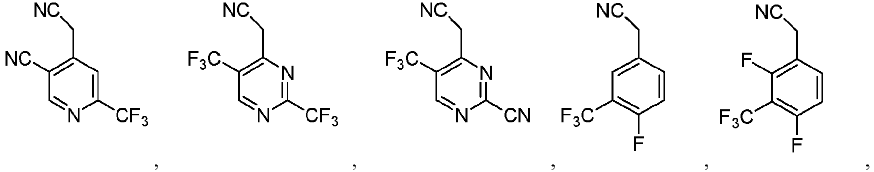 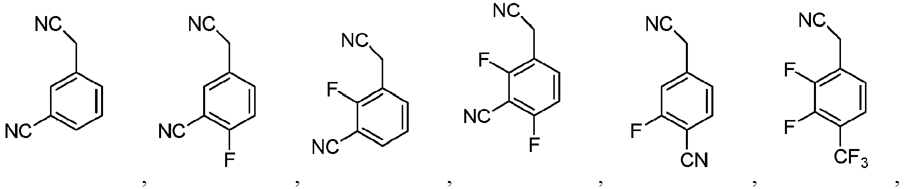 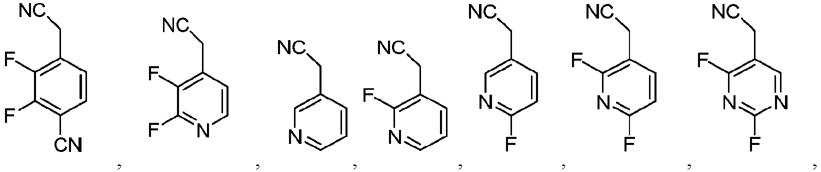 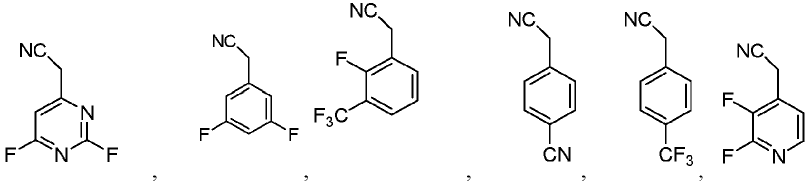 and

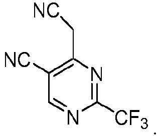

According to one embodiment of the present invention, formula (III) does not include the following moieties: 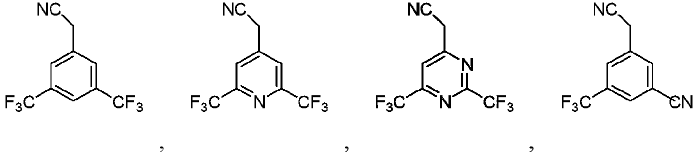 and

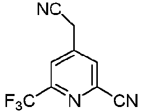

According to one embodiment of the present invention, the compound of formula (I) comprises less than nine CN groups, preferably less than eight CN groups.

According to one embodiment of the present invention, the compound of formula (I) comprises between three and eight CN groups, preferably between three and seven CN groups.

When the number of CN groups in the compound of formula (I) is selected in this range, improved processing properties, in particular in vacuum thermal deposition, may be obtained.

In the present invention, the LUMO level of compound of formula (I) is selected in the range of ≤ -4.7 eV and ≥ -5.25 eV when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase.

According to one embodiment of the present invention, the compound of formula (I) comprises between 3 and 8 CN groups, preferably between 3 and 7 CN groups, and the LUMO level of compound of formula (I) is selected in the range of ≤ -4.7 eV and ≥ -5.25 eV when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase.

According to one embodiment of the present invention, the LUMO level of compound of formula (I) is further away from vacuum level than 4,4',4"-((1E,1'E,1"E)-cyclopropane-1,2,3-triylidenetris(cyanomethaneylylidene))tribenzonitrile when determined under the same conditions, preferably further away from vacuum level than 4,4',4"-((1E,1'E,1"E)-cyclopropane-1,2,3-triylidenetris(cyanomethaneylylidene))tris(3-fluorobenzonitrile); more preferred further away from vacuum level than 4,4',4"-((1E,1'E,1"E)-cyclopropane-1,2,3-triylidenetris(cyanomethaneylylidene))tris(3-(trifluoromethyl)benzonitrile).

According to one embodiment of the present invention, the compound of formula (I) comprises between 3 and 8 CN groups, preferably between 3 and 7 CN groups, and the LUMO level of compound of formula (I) is further away from vacuum level than 4,4',4"-((1E,1'E,1"E)-cyclopropane-1,2,3-triylidenetris(cyanomethaneylylidene))tribenzonitrile when determined under the same conditions, preferably further away from vacuum level than 4,4',4"-((1E,1'E,1"E)-cyclopropane-1,2,3-triylidenetris(cyanomethaneylylidene))tris(3-fluorobenzonitrile); more preferred further away from vacuum level than 4,4',4"-((1E,1'E,1"E)-cyclopropane-1,2,3-triylidenetris(cyanomethaneylylidene))tris(3-(trifluoromethyl)benzonitrile).

According to one embodiment of the present invention, wherein in compound of formula (I) A¹, A² and A³ may be selected the same and the LUMO level of compound of formula (I) is selected in the range of ≤ -4.7 eV and ≥ -5.25 eV when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase.

According to one embodiment of the present invention, wherein the compound of formula (I) comprises between 3 and 8 CN groups, preferably between 3 and 7 CN groups, and in compound of formula (I) A¹, A² and A³ may be selected the same and the LUMO level of compound of formula (I) is selected in the range of ≤ -4.7 eV and ≥ -5.25 eV when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase.

According to one embodiment of the present invention, wherein in compound of formula (I) A¹ and A² may be selected the same or differently and A¹ is not identical to A³ and the LUMO level of compound of formula (I) is selected in the range of ≤ -4.7 eV and ≥ -5.25 eV when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase.

According to one embodiment of the present invention, wherein the compound of formula (I) comprises between 3 and 8 CN groups, preferably between 3 and 7 CN groups, and in compound of formula (I) A¹ and A² may be selected the same or differently and A¹ is not identical to A³ and the LUMO level of compound of formula (I) is selected in the range of ≤ -4.7 eV and ≥ -5.25 eV when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase.

According to one embodiment of the present invention, wherein in compound of formula (I) A¹, A² and A³ may be selected the same and the LUMO level of compound of formula (I) is selected in the range of ≤ -4.7 eV and ≥ -5.25 eV when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase; CN or N; preferably X², X³ and/or X⁴ are selected from CN or N and at least one remaining Rⁿ, wherein n is 2 to 5, is selected from halogen, Cl or F; or X², X³ and/or X⁴ are selected from perfluorinated C₁ to C₈ alkyl and at least one remaining Rⁿ, wherein n is an integer from 2 to 5, is selected from Cl or F; preferably at least two remaining Rⁿ, wherein n is an integer from 2 to 5, are selected from Cl or F.

According to one embodiment of the present invention, wherein the compound of formula (I) comprises between 3 and 8 CN groups, preferably between 3 and 7 CN groups, and in compound of formula (I) A¹, A² and A³ may be selected the same and the LUMO level of compound of formula (I) is selected in the range of ≤ -4.7 eV and ≥ -5.25 eV when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase; and X², X³ and/or X⁴ are selected from CN or N; preferably X², X³ and/or X⁴ are selected from CN or N and at least one remaining Rⁿ, wherein n is 2 to 5, is selected from Cl or F; or X², X³ and/or X⁴ are selected from perfluorinated C₁ to C₈ alkyl and at least one remaining Rⁿ, wherein n is an integer from 2 to 5, is selected from Cl or F; preferably at least two remaining Rⁿ, wherein n is an integer from 2 to 5, are selected from Cl or F.

According to one embodiment of the present invention, wherein in compound of formula (I) A¹ and A² may be selected the same or differently and A³ is not identical to A³ and the LUMO level of compound of formula (I) is selected in the range of ≤ -4.7 eV and ≥ -5.25 eV when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase; and X², X³ and/or X⁴ are selected from CN or N; preferably X², X³ and/or X⁴ are selected from CN or N and at least one remaining Rⁿ, wherein n is 2 to 5, is selected from Cl or F; or X², X³ and/or X⁴ are selected from perfluorinated C₁ to C₈ alkyl and at least one remaining Rⁿ, wherein n is an integer from 2 to 5, is selected from Cl or F; preferably at least two remaining Rⁿ, wherein n is an integer from 2 to 5, are selected from Cl or F.

According to one embodiment of the present invention, wherein the compound of formula (I) comprises between 3 and 8 CN groups, preferably between 3 and 7 CN groups, and in compound of formula (I) A¹ and A² may be selected the same or differently and A³ is not identical to A³ and the LUMO level of compound of formula (I) is selected in the range of ≤ -4.7 eV and ≥ -5.25 eV when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase; and X², X³ and/or X⁴ are selected from CN or N; preferably X², X³ and/or X⁴ are selected from CN or N and at least one remaining Rⁿ, wherein n is 2 to 5, is selected from Cl or F; or X², X³ and/or X⁴ are selected from perfluorinated C₁ to C₈ alkyl and at least one remaining Rⁿ, wherein n is an integer from 2 to 5, is selected from Cl or F; preferably at least two remaining Rⁿ, wherein n is an integer from 2 to 5, are selected from Cl or F

According to the present invention, wherein R' is CN , compound of formula (I) comprises between 3 and 8 CN groups, preferably between 3 and 7 CN groups; and the following formulas are excluded:

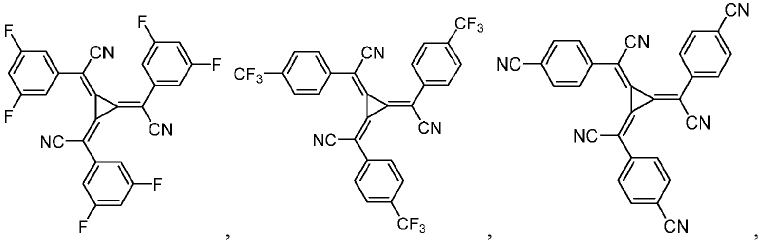

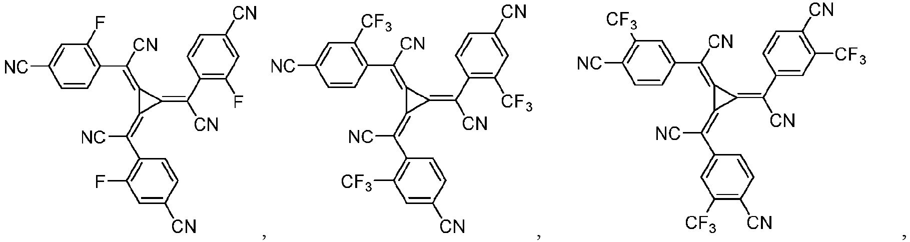

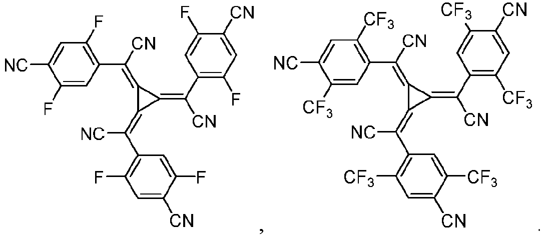

According to one embodiment, the compound of formula (I) is selected from the compounds A¹ to A101:

| | A¹ | A² | A³ |
|---|---|---|---|
| A1 | 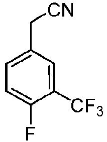 | 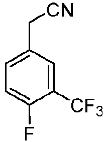 | 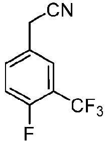 |
| A2 | 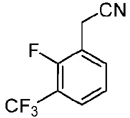 | 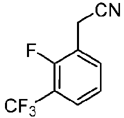 | 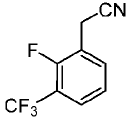 |
| A3 | 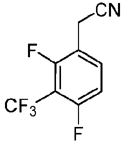 | 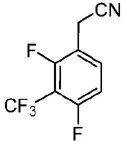 | 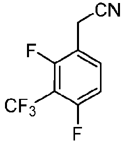 |
| A4 | 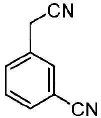 | 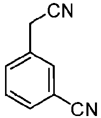 | 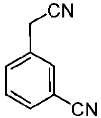 |
| A5 | 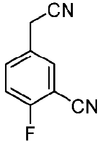 | 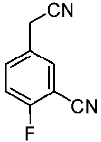 | 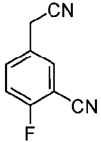 |
| A6 | 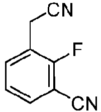 | 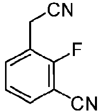 | 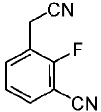 |
| A7 | 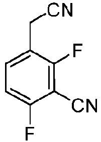 | 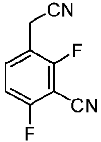 | 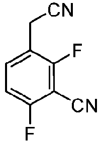 |
| A8 | 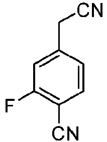 | 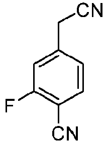 | 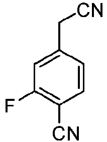 |
| A9 | 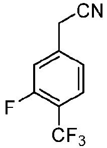 | 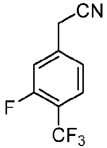 | 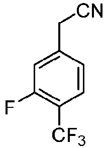 |
| A10 | 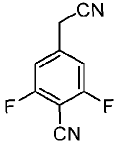 | 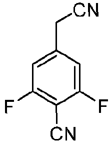 | 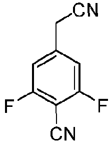 |
| A11 | 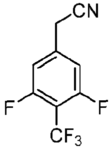 | 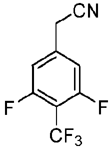 | 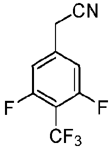 |
| A12 | 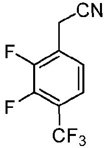 | 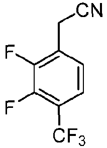 | 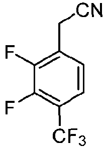 |
| A13 | 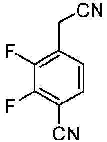 | 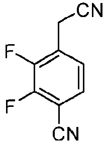 | 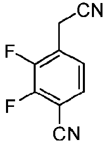 |
| A14 | 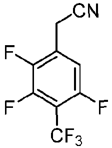 | 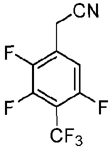 | 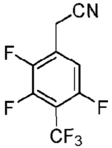 |
| A15 | 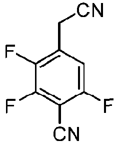 | 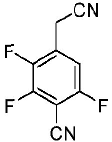 | 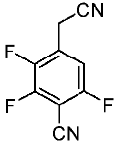 |
| A16 | 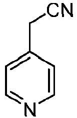 | 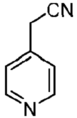 | 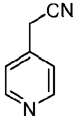 |
| A17 | 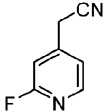 | 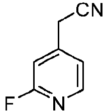 | 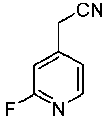 |
| A18 | 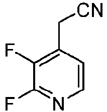 | 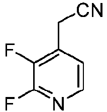 | 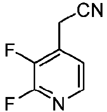 |
| A19 | 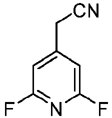 | 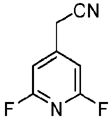 | 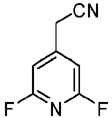 |
| A20 | 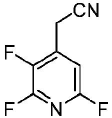 | 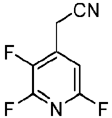 | 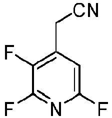 |
| A21 | 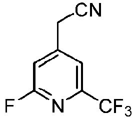 | 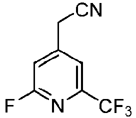 | 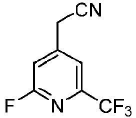 |
| A22 | 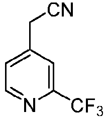 | 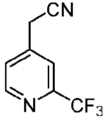 | 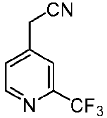 |
| A23 | 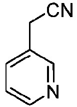 | 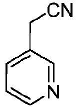 | 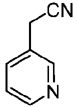 |
| A24 | 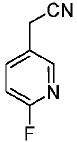 | 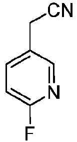 | 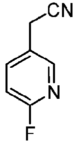 |
| A25 | 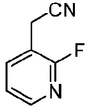 | 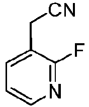 | 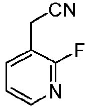 |
| A26 | 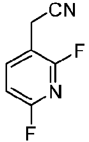 | 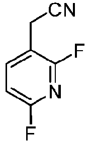 | 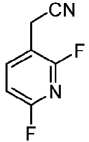 |
| A27 | 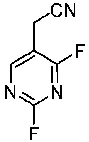 | 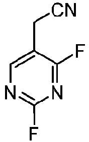 | 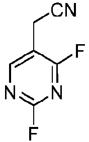 |
| A28 | 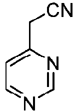 | 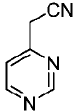 | 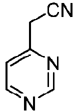 |
| A29 | 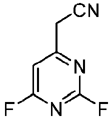 | 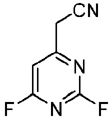 | 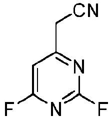 |
| A30 | 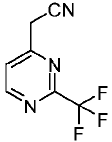 | 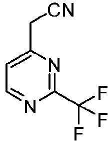 | 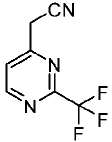 |
| A31 | 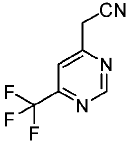 | 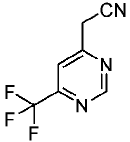 | 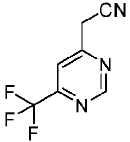 |
| A32 | 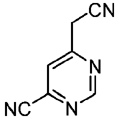 | 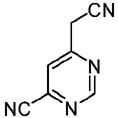 | 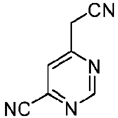 |
| A33 | 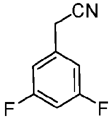 | 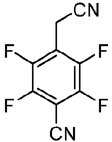 | 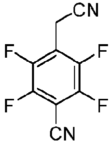 |
| A34 | 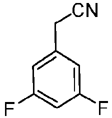 | 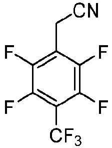 | 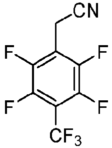 |
| A35 | 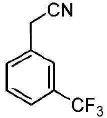 | 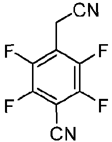 | 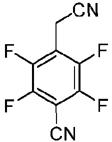 |
| A36 | 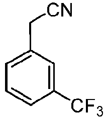 | 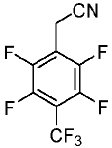 | 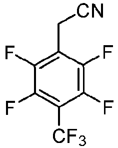 |
| A37 | 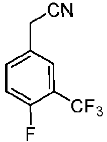 | 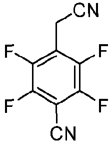 | 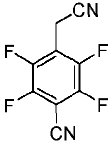 |
| A38 | 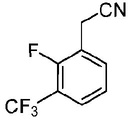 | 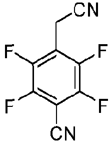 | 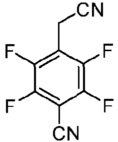 |
| A39 | 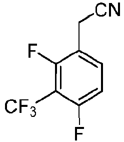 | 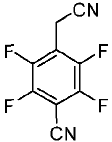 | 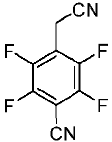 |
| A40 | 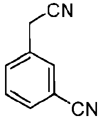 | 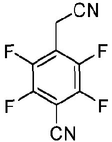 | 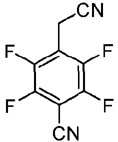 |
| A41 | 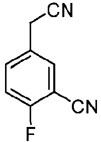 | 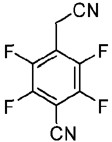 | 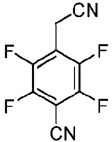 |
| A42 | 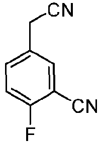 | 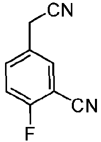 | 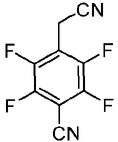 |
| A43 | 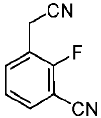 | 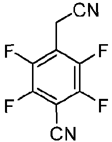 | 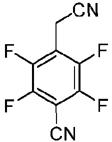 |
| A44 | 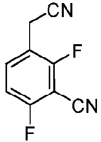 | 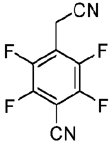 | 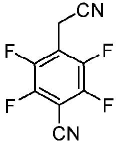 |
| A45 | 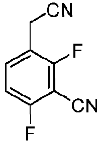 | 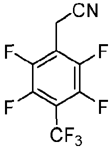 | 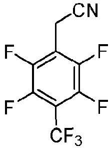 |
| A46 | 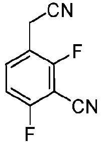 | 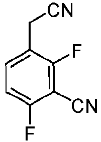 | 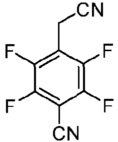 |
| A47 | 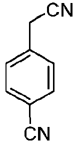 | 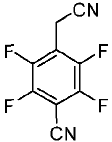 | 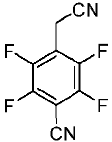 |
| A48 | 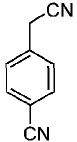 | 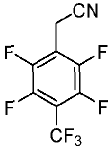 | 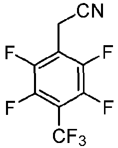 |
| A49 | 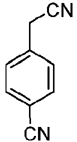 | 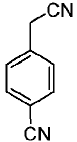 | 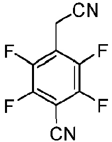 |
| A50 | 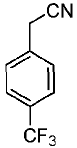 | 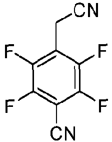 | 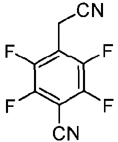 |
| A51 | 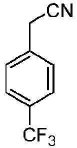 | 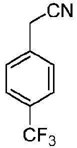 | 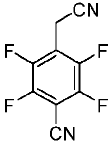 |
| A52 | 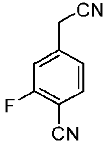 | 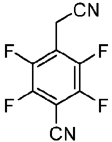 | 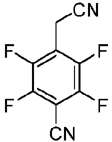 |
| A53 | 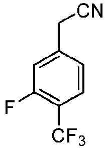 | 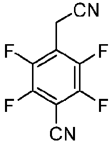 | 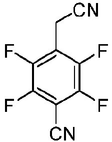 |
| A54 | 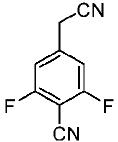 | 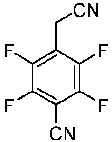 | 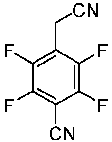 |
| A55 | 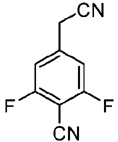 | 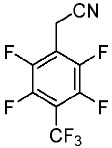 | 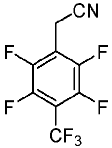 |
| A56 | 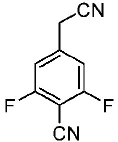 | 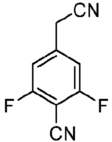 | 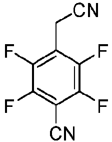 |
| A57 | 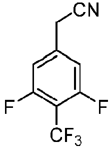 | 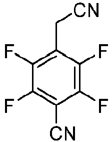 | 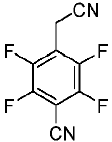 |
| A58 | 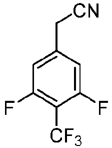 | 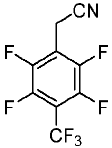 | 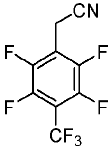 |
| A59 | 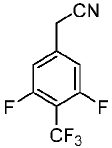 | 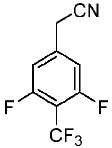 | 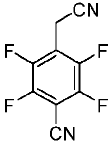 |
| A60 | 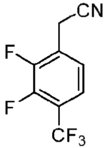 | 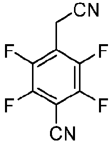 | 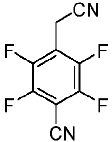 |
| A61 | 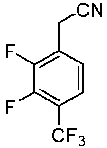 | 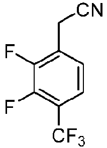 | 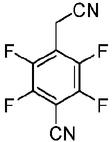 |
| A62 | 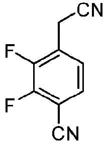 | 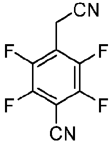 | 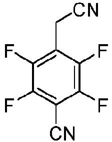 |
| A63 | 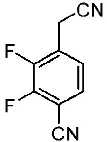 | 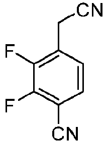 | 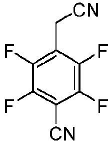 |
| A64 | 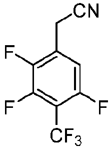 | 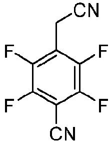 | 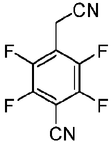 |
| A65 | 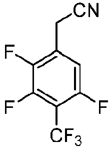 | 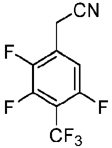 | 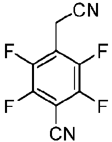 |
| A66 | 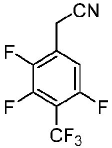 | 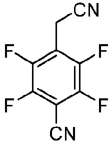 | 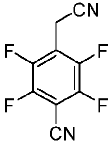 |
| A67 | 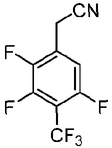 | 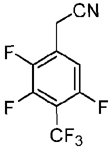 | 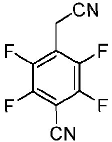 |
| A68 | 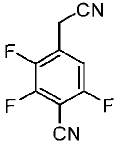 | 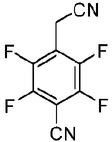 | 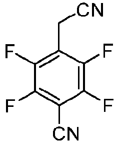 |
| A69 | 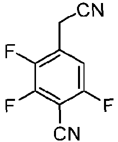 | 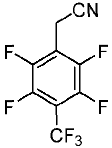 | 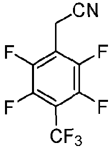 |
| A70 | 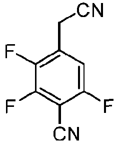 | 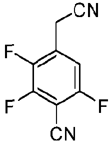 | 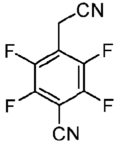 |
| A71 | 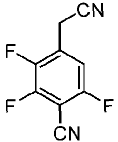 | 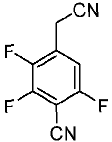 | 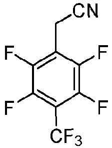 |
| A72 | 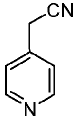 | 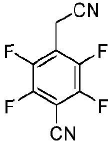 | 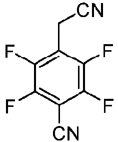 |
| A73 | 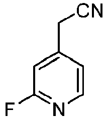 | 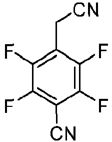 | 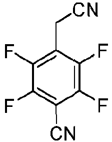 |
| A74 | 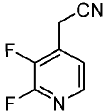 | 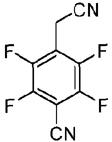 | 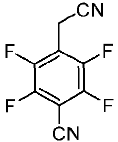 |
| A75 | 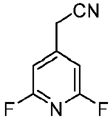 | 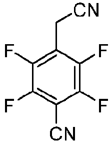 | 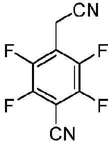 |
| A76 | 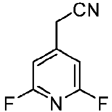 | 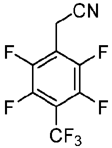 | 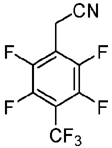 |
| A77 | 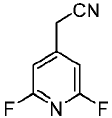 | 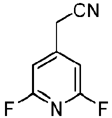 | 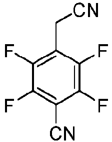 |
| A78 | 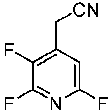 | 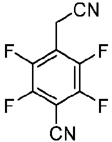 | 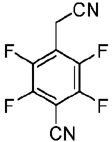 |
| A79 | 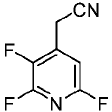 | 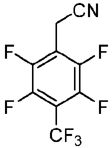 | 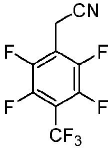 |
| A80 | 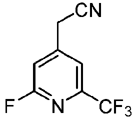 | 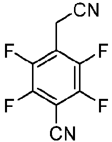 | 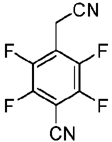 |
| A81 | 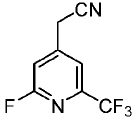 | 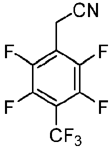 | 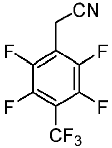 |
| A82 | 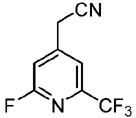 | 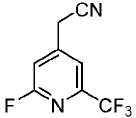 | 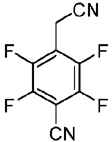 |
| A83 | 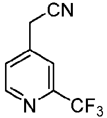 | 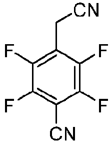 | 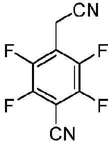 |
| A84 | 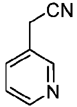 | 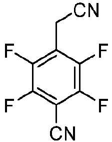 | 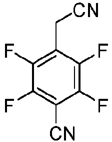 |
| A85 | 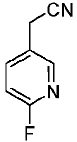 | 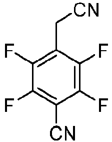 | 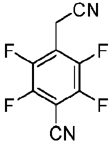 |
| A86 | 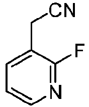 | 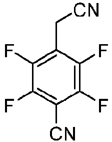 | 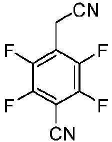 |
| A87 | 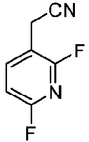 | 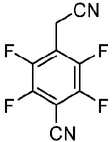 | 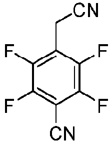 |
| A88 | 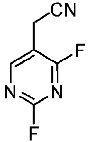 | 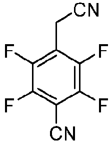 | 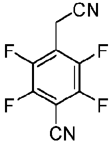 |
| A89 | 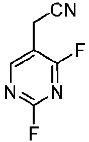 | 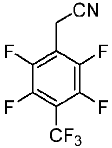 | 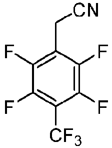 |
| A90 | 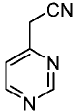 | 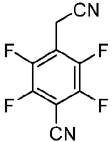 | 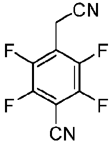 |
| A91 | 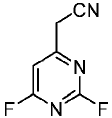 | 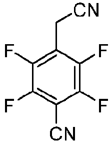 | 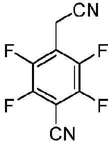 |
| A92 | 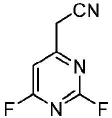 | 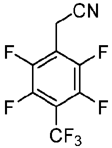 | 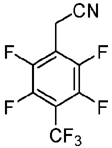 |
| A93 | 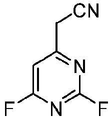 | 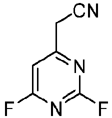 | 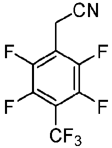 |
| A94 | 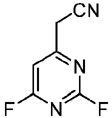 | 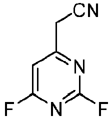 | 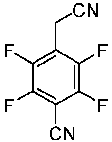 |
| A95 | 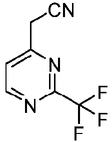 | 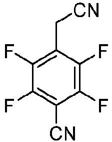 | 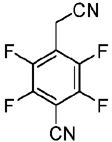 |
| A96 | 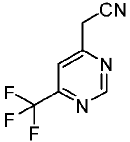 | 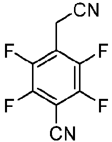 | 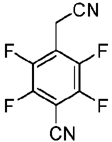 |
| A97 | 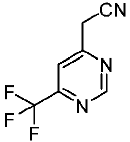 | 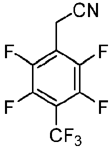 | 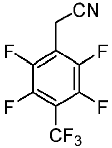 |
| A98 | 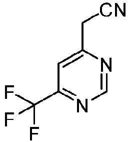 | 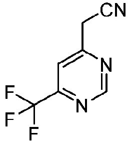 | 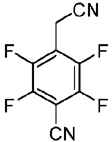 |
| A99 | 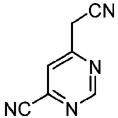 | 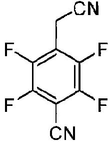 | 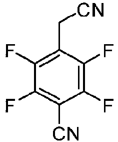 |
| A100 | 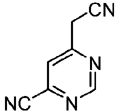 | 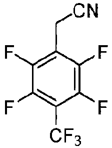 | 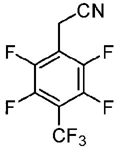 |
| A101 | 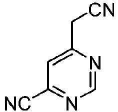 | 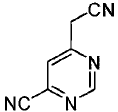 | 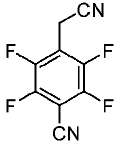 |

The present invention furthermore relates to an organic electronic device comprising an anode layer, a cathode layer and at least one organic semiconductor layer, wherein the at least one organic semiconductor layer is arranged between the anode layer and the cathode layer; and wherein the at least one organic semiconductor layer comprises a compound of formula (I).

According to one embodiment, the organic semiconductor layer comprises a composition comprising a compound of formula (IV) and at least one compound of formula (IVa) to (IVd)

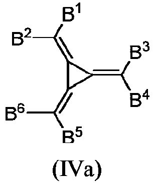

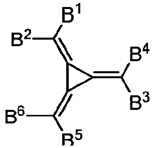

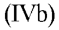

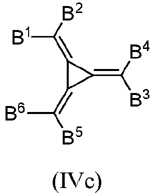

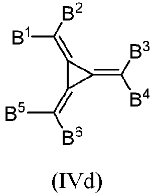

In case the organic semiconductor layer comprises such a composition, throughout this application text the term "compound of formula (I)" shall also intend to include the composition as described above.

According to one embodiment of the present invention the organic semiconductor layer and/or the compound of formula (I) are non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

According to one embodiment of the invention, the at least one organic semiconductor layer further comprises a substantially covalent matrix compound.

### Substantially covalent matrix compound

The organic semiconductor layer may further comprises a substantially covalent matrix compound. According to one embodiment the substantially covalent matrix compound may be selected from at least one organic compound. The substantially covalent matrix may consists substantially from covalently bound C, H, O, N, S, which optionally comprise in addition covalently bound B, P, As and/or Se.

According to one embodiment of the organic electronic device, the organic semiconductor layer further comprises a substantially covalent matrix compound, wherein the substantially covalent matrix compound may be selected from organic compounds consisting substantially from covalently bound C, H, O, N, S, which optionally comprise in addition covalently bound B, P, As and/or Se.

Organometallic compounds comprising covalent bonds carbon-metal, metal complexes comprising organic ligands and metal salts of organic acids are further examples of organic compounds that may serve as substantially covalent matrix compounds of the hole injection layer.

In one embodiment, the substantially covalent matrix compound lacks metal atoms and majority of its skeletal atoms may be selected from C, O, S, N. Alternatively, the substantially covalent matrix compound lacks metal atoms and majority of its skeletal atoms may be selected from C and N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

Preferably, the substantially covalent matrix compound comprises at least one arylamine moiety, alternatively a diarylamine moiety, alternatively a triarylamine moiety.

Preferably, the substantially covalent matrix compound is free of metals and/or ionic bonds.

### Compound of formula (VI) or a compound of formula (VII)

According to another aspect of the present invention, the at least one matrix compound, also referred to as "substantially covalent matrix compound", may comprises at least one arylamine compound, diarylamine compound, triarylamine compound, a compound of formula (VI) or a compound of formula (VII): 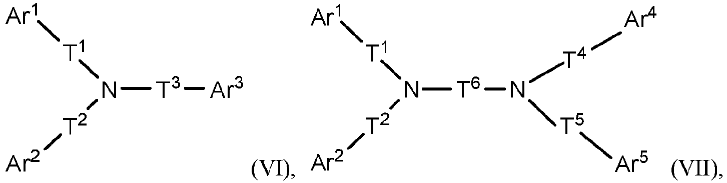 wherein:
T¹, T², T³, T⁴ and T⁵ are independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein
the substituents of Ar¹, Ar², Ar³, Ar⁴ and Ar⁵ are selected the same or different from the group comprising H, D, F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

According to an embodiment wherein T¹, T², T³, T⁴ and T⁵ may be independently selected from a single bond, phenylene, biphenylene or terphenylene. According to an embodiment wherein T¹, T², T³, T⁴ and T⁵ may be independently selected from phenylene, biphenylene or terphenylene and one of T¹, T², T³, T⁴ and T⁵ are a single bond. According to an embodiment wherein T¹, T², T³, T⁴ and T⁵ may be independently selected from phenylene or biphenylene and one of T¹, T², T³, T⁴ and T⁵ are a single bond. According to an embodiment wherein T¹, T², T³, T⁴ and T⁵ may be independently selected from phenylene or biphenylene and two of T¹, T², T³, T⁴ and T⁵ are a single bond.

According to an embodiment wherein T¹, T² and T³ may be independently selected from phenylene and one of T¹, T² and T³ are a single bond. According to an embodiment wherein T¹, T² and T³ may be independently selected from phenylene and two of T¹, T² and T³ are a single bond.

According to an embodiment wherein T⁶ may be phenylene, biphenylene, terphenylene. According to an embodiment wherein T⁶ may be phenylene. According to an embodiment wherein T⁶ may be biphenylene. According to an embodiment wherein T⁶ may be terphenylene.

According to an embodiment wherein Ar¹, Ar², Ar³, Ar⁴ and Ar⁵ may be independently selected from D1 to D16: 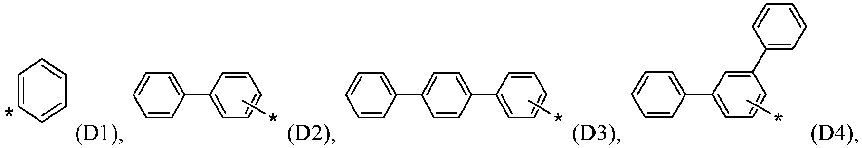 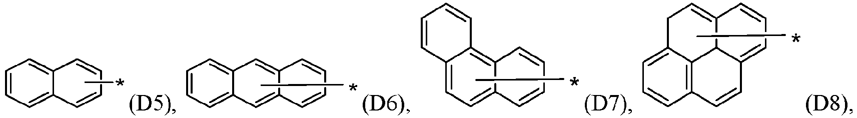 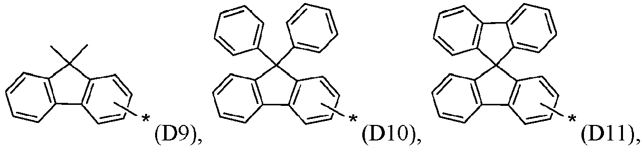 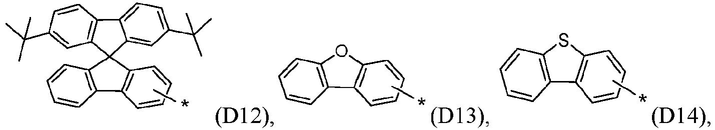 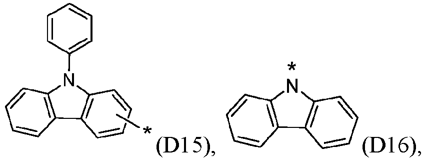 wherein the asterix "*" denotes the binding position.

According to an embodiment, wherein Ar¹, Ar², Ar³, Ar⁴ and Ar⁵ may be independently selected from D1 to D15; alternatively selected from D1 to D10 and D13 to D15.

According to an embodiment, wherein Ar¹, Ar², Ar³, Ar⁴ and Ar⁵ may be independently selected from the group consisting of D1, D2, D5, D7, D9, D10, D13 to D16.

The rate onset temperature may be in a range particularly suited to mass production, when Ar¹, Ar², Ar³, Ar⁴ and Ar⁵ are selected in this range.

The "matrix compound of formula (VI) or formula (VII)" may be also referred to as "hole transport compound".

According to one embodiment, the substantially covalent matrix compound comprises at least one naphthyl group, carbazole group, dibenzofurane group, dibenzothiophene group and/or substituted fluorenyl group, wherein the substituents are independently selected from methyl, phenyl or fluorenyl.

According to an embodiment of the electronic device, wherein the matrix compound of formula (VI) or formula (VII) are selected from F1 to F19:

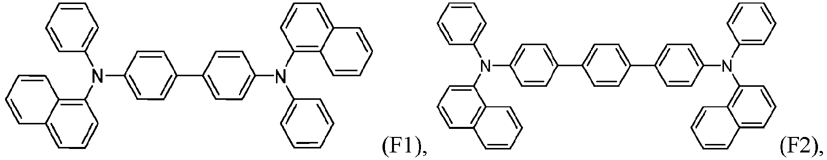

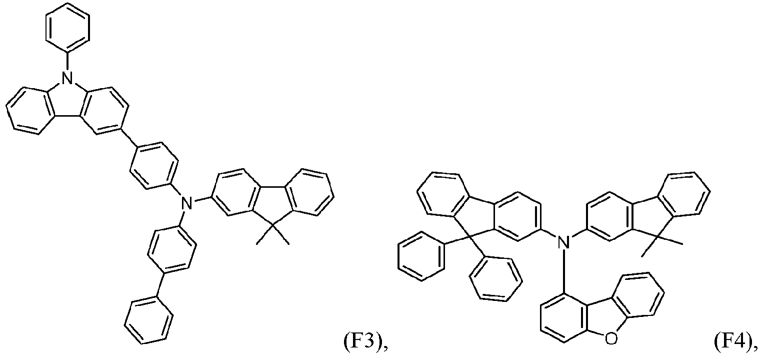

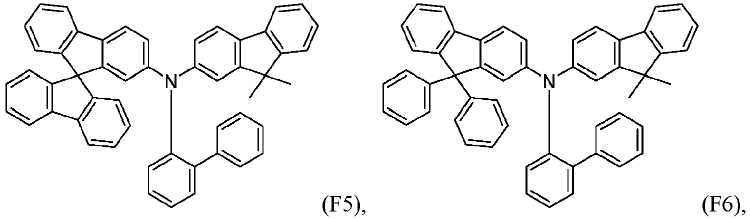

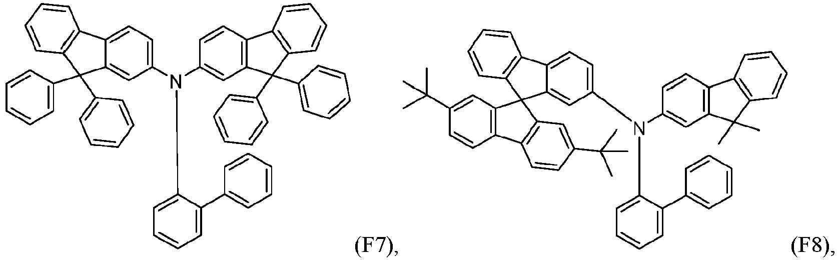

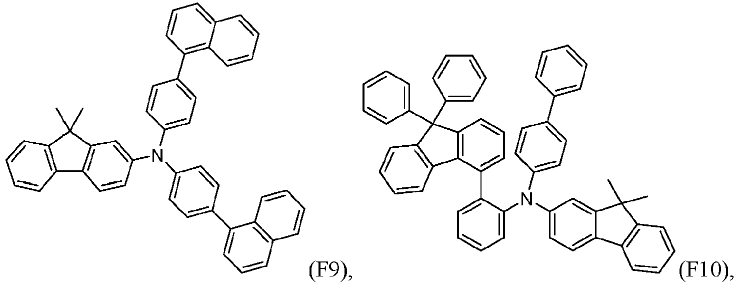

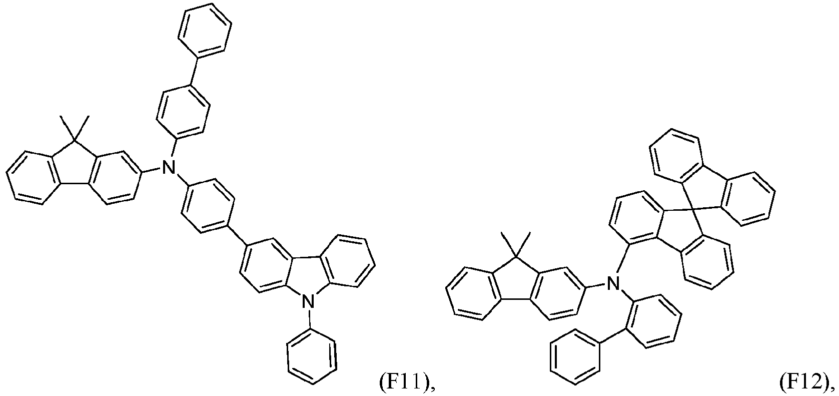

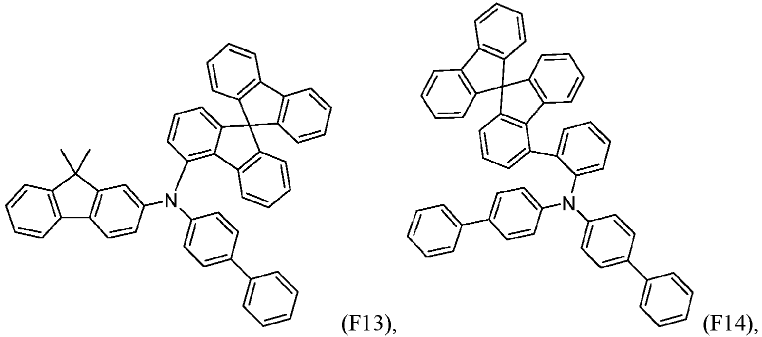

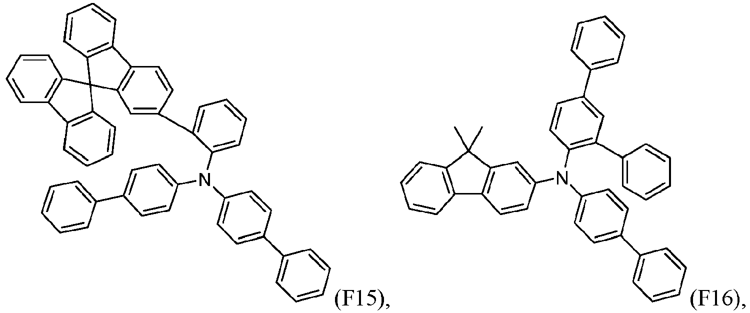

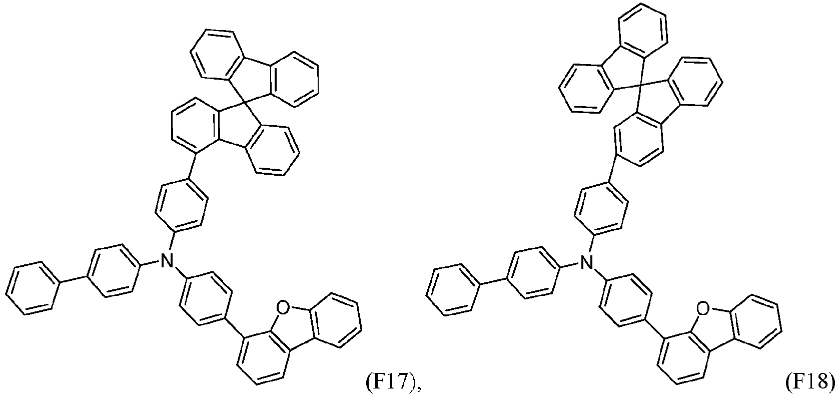

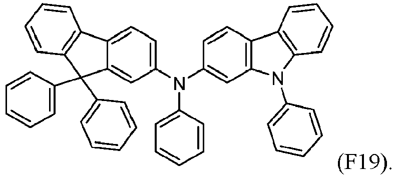

### Organic semiconductor layer

The organic semiconductor layer may be formed on the anode layer or cathode layer by vacuum deposition, spin coating, printing, casting, slot-die coating, Langmuir-Blodgett (LB) deposition, or the like. When the Organic semiconductor layer is formed using vacuum deposition, the deposition conditions may vary according to the compound(s) that are used to form the layer, and the desired structure and thermal properties of the layer. In general, however, conditions for vacuum deposition may include a deposition temperature of 100° C to 350° C, a pressure of 10⁻⁸ to 10⁻³ Torr (1 Torr equals 133.322 Pa), and a deposition rate of 0.1 to 10 nm/sec.

When the organic semiconductor layer is formed using spin coating or printing, coating conditions may vary according to the compound(s) that are used to form the layer, and the desired structure and thermal properties of the organic semiconductor layer. For example, the coating conditions may include a coating speed of about 2000 rpm to about 5000 rpm, and a thermal treatment temperature of about 80° C to about 200° C. Thermal treatment removes a solvent after the coating is performed.

The thickness of the organic semiconductor layer may be in the range from about 1 nm to about 20 nm, and for example, from about 2 nm to about 15 nm, alternatively about 2 nm to about 12 nm.

When the thickness of the organic semiconductor layer is within this range, the organic semiconductor layer may have excellent hole injecting and/or hole generation characteristics, without a substantial penalty in driving voltage.

According to one embodiment of the present invention, the organic semiconductor layer may comprise:
- at least about ≥ 0.5 wt.-% to about ≤ 30 wt.-%, preferably about ≥ 0.5 wt.-% to about ≤ 20 wt.-%, and more preferred about ≥ 1 wt.-% to about ≤ 15 wt.-% of a compound of formula (I), and
- at least about ≥ 70 wt.-% to about ≤ 99.5 wt.-%, preferably about ≥ 80 wt.-% to about ≤ 99.5 wt.-%, and more preferred about ≥ 85 wt.-% to about ≤ 99 wt.-% of a substantially covalent matrix compound; preferably the wt.-% of the compound of formula (I) is lower than the wt.-% of the substantially covalent matrix compound; wherein the weight-% of the components are based on the total weight of the organic semiconductor layer.

According to one embodiment of the invention, the organic electronic device comprises at least one photoactive layer and the at least one of the at least one organic semiconductor layers is arranged between the anode and the at least one photoactive layer.

According to one embodiment of the invention, the organic electronic device comprises at least one photoactive layer and the at least one organic semiconductor layers is arranged between the anode and the at least one photoactive layer.

According to one embodiment of the invention, the organic electronic device comprises at least two photoactive layers, wherein at least one of the at least one organic semiconductor layers is arranged between the first and the second photoactive layer.

According to one embodiment of the invention, the organic electronic devices comprises at least one photoactive layer, wherein the photoactive layer is arranged between the anode layer and the cathode layer.

According to one embodiment of the invention, the organic electronic devices comprises at least one photoactive layer and at least one of the at least one organic semiconductor layers is arranged between the anode and the at least one photoactive layer.

According to one embodiment of the invention, the organic electronic devices comprises at least one photoactive layer and the at least one organic semiconductor layers is arranged between the anode and the at least one photoactive layer.

According to one embodiment of the invention, the organic electronic device comprises at least two photoactive layers, wherein at least one of the at least one organic semiconductor layers is arranged between the first and the second photoactive layer.

According to one embodiment of the invention, the organic electronic device comprises at least two photoactive layers, wherein one of the at least one organic semiconductor layers is arranged between the first and the second photoactive layer and one of the at least one organic semiconductor layers is arranged between the anode layer and the first photoactive layer.

According to one embodiment of the invention the electronic organic device is an electroluminescent device, preferably an organic light emitting diode.

According to one embodiment of the invention, the electronic organic device is not an organic light emitting diode comprising a substrate, an anode, a cathode, a first emission layer, an electron injection layer and a second electron transport layer stack, wherein the second electron transport layer stack is arranged between the first emission layer and the electron injection layer; wherein
- at least one of the first electron transport layer stack and the second electron transport layer stack comprises independently a first electron transport layer and a second electron transport layer;
- the first electron transport layer comprises a compound of Formula (X)

   (Ar¹-A_{c})ₐ-X_{b} (X);
- a and b are independently 1 or 2;
- c is independently 0 or 1;
- Ar¹ is independently selected from C₆ to C₆₀ aryl or C₂ to C₄₂ heteroaryl,
- wherein each Ar¹ may be substituted with one or two substituents independently selected from the group consisting of C₆ to C₁₂ aryl, C₃ to C₁₁ heteroaryl, and C₁ to C₆ alkyl, D, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₆ alkyl, partially or perfluorinated C₁ to C₆ alkoxy, partially or perdeuterated C₁ to C₆ alkyl, partially or perdeuterated C₁ to C₆ alkoxy, halogen, CN or PY(R¹⁰)₂, wherein Y is selected from O, S or Se, preferably O and R¹⁰ is independently selected from C₆ to C₁₂ aryl, C₃ to C₁₂ heteroaryl, C₁ to C₆ alkyl, C₁ to C₆ alkoxy, partially or perfluorinated C₁ to C₆ alkyl, partially or perfluorinated C₁ to C₆ alkoxy, partially or perdeuterated C₁ to C₆ alkyl, partially or perdeuterated C₁ to C₆ alkoxy;
- wherein each C₆ to C₁₂ aryl substituent on Ar¹ and each C₃ to C₁₁ heteroaryl substituent on Ar¹ may be substituted with C₁ to C₄ alkyl or halogen;
- A is independently selected from C₆ to C₃₀ aryl,
- wherein each A may be substituted with one or two substituents independently selected from the group consisting of C₆ to C₁₂ aryl and C₁ to C₆ alkyl, D, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₆ alkyl, partially or perfluorinated C₁ to C₆ alkoxy, partially or perdeuterated C₁ to C₆ alkyl, partially or perdeuterated C₁ to C₆ alkoxy, halogen, CN or PY(R¹⁰)₂, wherein Y is selected from O, S or Se, preferably O, and R¹⁰ is independently selected from C₆ to C₁₂ aryl, C₃ to C₁₂ heteroaryl, C₁ to C₆ alkyl, C₁ to C₆ alkoxy, partially or perfluorinated C₁ to C₆ alkyl, partially or perfluorinated C₁ to C₆ alkoxy, partially or perdeuterated C₁ to C₆ alkyl, partially or perdeuterated C₁ to C₆ alkoxy;
- wherein each C₆ to C₁₂ aryl substituent on A may be substituted with C₁ to C₄ alkyl or halogen;
- X is independently selected from the group consisting of C₂ to C₄₂ heteroaryl and C₆ to C₆₀ aryl,
- wherein each X may be substituted with one or two substituents independently selected from the group consisting of C₆ to C₁₂ aryl, C₃ to C₁₁ heteroaryl, and C₁ to C₆ alkyl, D, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₆ alkyl, partially or perfluorinated C₁ to C₆ alkoxy, partially or perdeuterated C₁ to C₆ alkyl, partially or perdeuterated C₁ to C₆ alkoxy, halogen, CN or PY(R¹⁰)₂, wherein Y is selected from O, S or Se, preferably O, and R¹⁰ is independently selected from C₆ to C₁₂ aryl, C₃ to C₁₂ heteroaryl, C₁ to C₆ alkyl, C₁ to C₆ alkoxy, partially or perfluorinated C₁ to C₆ alkyl, partially or perfluorinated C₁ to C₆ alkoxy, partially or perdeuterated C₁ to C₆ alkyl, partially or perdeuterated C₁ to C₆ alkoxy;
- wherein each C₆ to C₁₂ aryl substituent on X and each C₃ to C₁₁ heteroaryl substituent on X may be substituted with C₁ to C₄ alkyl or halogen;
- the molecular dipole moment of the compound of formula (X) is ≥ 0 D and ≤ 4 D;
- the second electron transport layer comprises a compound of Formula (XI)

   (Ar²)ₘ-(Zₖ-G)ₙ (XI);
- m and n are independently 1 or 2;
- k is independently 0, 1 or 2;
- Ar² is independently selected from the group consisting of C₂ to C₄₂ heteroaryl and C₆ to C₆₀ aryl,
- wherein each Ar² may be substituted with one or two substituents independently selected from the group consisting of C₆ to C₁₂ aryl, C₃ to C₁₁ heteroaryl, and C₁ to C₆ alkyl, D, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₆ alkyl, partially or perfluorinated C₁ to C₆ alkoxy, partially or perdeuterated C₁ to C₆ alkyl, partially or perdeuterated C₁ to C₆ alkoxy, halogen, CN or PY(R¹⁰)₂, wherein Y is selected from O, S or Se, preferably O, and R¹⁰ is independently selected from C₆ to C₁₂ aryl, C₃ to C₁₂ heteroaryl, C₁ to C₆ alkyl, C₁ to C₆ alkoxy, partially or perfluorinated C₁ to C₆ alkyl, partially or perfluorinated C₁ to C₆ alkoxy, partially or perdeuterated C₁ to C₆ alkyl, partially or perdeuterated C₁ to C₆ alkoxy;
- wherein each C₆ to C₁₂ aryl substituent on Ar² and each C₃ to C₁₁ heteroaryl substituent on Ar² may be substituted with C₁ to C₄ alkyl or halogen;
- Z is independently selected from C₆ to C₃₀ aryl,
- wherein each Z may be substituted with one or two substituents independently selected from the group consisting of C₆ to C₁₂ aryl and C₁ to C₆ alkyl, D, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₆ alkyl, partially or perfluorinated C₁ to C₆ alkoxy, partially or perdeuterated C₁ to C₆ alkyl, partially or perdeuterated C₁ to C₆ alkoxy, halogen, CN or PY(R¹⁰)₂, wherein Y is selected from O, S or Se, preferably O, and R¹⁰ is independently selected from C₆ to C₁₂ aryl, C₃ to C₁₂ heteroaryl, C₁ to C₆ alkyl, C₁ to C₆ alkoxy, partially or perfluorinated C₁ to C₆ alkyl, partially or perfluorinated C₁ to C₆ alkoxy, partially or perdeuterated C₁ to C₆ alkyl, partially or perdeuterated C₁ to C₆ alkoxy;
- wherein each C₆ to C₁₂ aryl substituent on Z may be substituted with C₁ to C₄ alkyl or halogen;
- G is chosen so that the dipole moment of a compound G-phenyl is ≥ 1 D and ≤ 7 D; and
- the first electron transport layer and the second electron transport layer are free of an electrical dopant;
characterized in that
- the organic light emitting diode further comprises a p-type layer;
- the p-type layer is arranged between the anode and the first emission layer; and
- the p-type layer comprises a radialene compound.

The present invention furthermore relates to an organic electronic device comprising an anode layer, a cathode layer, at least one photoactive layer and at least one organic semiconductor layer, wherein the at least one organic semiconductor layer is arranged between the at least one photoactive layer and the cathode ; and wherein the at least one organic semiconductor layer comprises a compound of the present invention.

According to one embodiment of the present invention, the organic electronic device comprises at least one photoactive layer, wherein the at least one photoactive layer is arranged between the anode layer and the cathode layer and at least one of the at least one organic semiconductor layers is arranged between the anode layer and the at least one photoactive layer.

The present invention furthermore relates to a display device comprising an organic electronic device according to the present invention.

### Further layers

In accordance with the invention, the organic electronic device may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

### Anode layer

The anode layer may be formed by depositing or sputtering a material that is used to form the anode layer. The material used to form the anode layer may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode layer may also be formed using metals, typically silver (Ag), gold (Au), or metal alloys.

### Hole injection layer

A hole injection layer (HIL) may be formed on the anode layer by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10⁻⁸ to 10⁻³ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

### Hole transport layer

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to one embodiment of the present invention, the hole transport layer may comprise the same substantially covalent matrix compound as the organic semiconductor layer of the present invention.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

### Photoactive layer (PAL)

The photoactive layer converts an electrical current into photons or photons into an electrical current.

The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

According to one embodiment of the present invention, the photoactive layer does not comprise the compound of formula (I).

The photoactive layer may be a light-emitting layer or a light-absorbing layer.

### Emission layer (EML)

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

According to one embodiment of the present invention, the emission layer does not comprise the compound of formula (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and azine derivatives, preferably triazine or pyrimidine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The organic electronic device according to the present invention may further comprise an electron transport layer (ETL).

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

### Electron injection layer (EIL)

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Cathode layer

The cathode layer is formed on the ETL or optional EIL. The cathode layer may be formed of a metal, an alloy, an electrically conductive compound, or a mixture thereof. The cathode electrode may have a low work function For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

It is to be understood that the cathode layer is not part of an electron injection layer or the electron transport layer.

### Organic light-emitting diode (OLED)

The organic electronic device according to the invention may be an organic light-emitting device.

According to one aspect of the present invention, there is provided an organic light-emitting diode (OLED) comprising: a substrate; an anode electrode formed on the substrate; an organic semiconductor layer comprising a compound of formula (I) , a hole transport layer, an emission layer, an electron transport layer and a cathode electrode.

According to another aspect of the present invention, there is provided an OLED comprising: a substrate; an anode electrode formed on the substrate; an organic semiconductor layer comprising a compound of formula (I), a hole transport layer, an electron blocking layer, an emission layer, a hole blocking layer, an electron transport layer and a cathode electrode.

According to another aspect of the present invention, there is provided an OLED comprising: a substrate; an anode electrode formed on the substrate; an organic semiconductor layer comprising a compound of formula (I), a hole transport layer, an electron blocking layer, an emission layer, a hole blocking layer, an electron transport layer, an electron injection layer, and a cathode electrode.

According to various embodiments of the present invention, there may be provided OLEDs layers arranged between the above mentioned layers, on the substrate or on the top electrode.

According to one aspect, the OLED may comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

The organic semiconductor layer according to the invention may be the first hole injection layer and/or the p-type charge generation layer.

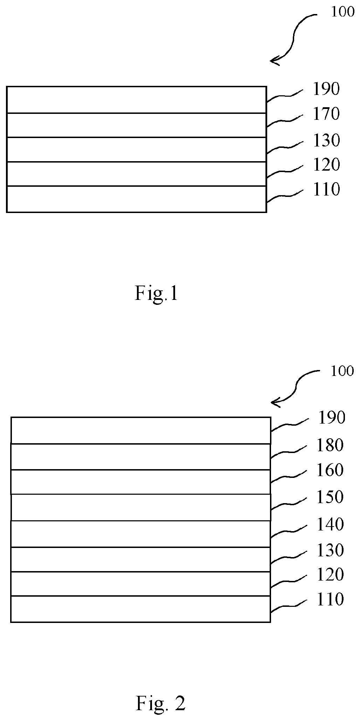

For example, the OLED according to Fig. 2 may be formed by a process, wherein on a substrate (110), an anode layer (120), a hole injection layer (130), a hole transport layer (140), an electron blocking layer (145), an emission layer (150), a hole blocking layer (155), an electron transport layer (160), an electron injection layer (180) and the cathode layer (190) are subsequently formed in that order.

### Organic electronic device

The organic electronic device according to the invention may be a light emitting device, or a photovoltaic cell, and preferably a light emitting device.

According to another aspect of the present invention, there is provided a method of manufacturing an organic electronic device, the method using:
- at least one deposition source, preferably two deposition sources and more preferred at least three deposition sources.

The methods for deposition that can be suitable comprise:
- deposition via vacuum thermal evaporation;
- deposition via solution processing, preferably the processing is selected from spin-coating, printing, casting; and/or
- slot-die coating.

According to various embodiments of the present invention, there is provided a method using:
- a first deposition source to release the compound of formula (I) according to the invention, and
- a second deposition source to release the substantially covalent matrix compound; the method comprising the steps of forming the organic semiconductor layer; whereby for an organic light-emitting diode (OLED):
- the organic semiconductor layer is formed by releasing the compound of formula (I) according to the invention from the first deposition source and the substantially covalent matrix compound from the second deposition source.

According to various embodiments of the present invention, the method may further include forming on the anode electrode, at least one layer selected from the group consisting of forming a hole transport layer or forming a hole blocking layer, and an emission layer between the anode electrode and the first electron transport layer.

According to various embodiments of the present invention, the method may further include the steps for forming an organic light-emitting diode (OLED), wherein
- on a substrate an anode electrode is formed,
- on the anode electrode an organic semiconductor layer comprising a compound of formula (I) is formed,
- on the organic semiconductor layer comprising a compound of formula (I) a hole transport layer is formed,
- on the hole transport layer an emission layer is formed,
- on the emission layer an electron transport layer is formed, optionally a hole blocking layer is formed on the emission layer,
- and finally a cathode electrode is formed,
- optional a hole blocking layer is formed in that order between the first anode electrode and the emission layer,
- optional an electron injection layer is formed between the electron transport layer and the cathode electrode.

According to various embodiments, the OLED may have the following layer structure, wherein the layers having the following order:
anode, organic semiconductor layer comprising a compound of formula (I) according to the invention, first hole transport layer, second hole transport layer, emission layer, optional hole blocking layer, electron transport layer, optional electron injection layer, and cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.
FIG. 1 is a schematic sectional view of an organic electronic device, according to an exemplary embodiment of the present invention;
FIG. 2 is a schematic sectional view of an organic light-emitting diode (OLED), according to an exemplary embodiment of the present invention;
FIG. 3 is a schematic sectional view of an OLED, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic electronic device 100, according to an exemplary embodiment of the present invention. The organic electronic device 100 includes a substrate 110, an anode layer 120 and a hole injection layer (HIL) (130) which may comprise a compound of formula (I). The HIL 130 is disposed on the anode layer 120. Onto the HIL 130, a photoactive layer (PAL) 170 and a cathode layer 190 are disposed.

FIG. 2 is a schematic sectional view of an organic light-emitting diode (OLED) 100, according to an exemplary embodiment of the present invention. The OLED 100 includes a substrate 110, an anode layer 120 and a hole injection layer (HIL) 130 which may comprise a compound of formula (I). The HIL 130 is disposed on the anode layer 120. Onto the HIL 130, a hole transport layer (HTL) 140, an emission layer (EML) 150, an electron transport layer (ETL) 160, an electron injection layer (EIL) 180 and a cathode layer 190 are disposed. Instead of a single electron transport layer 160, optionally an electron transport layer stack (ETL) can be used.

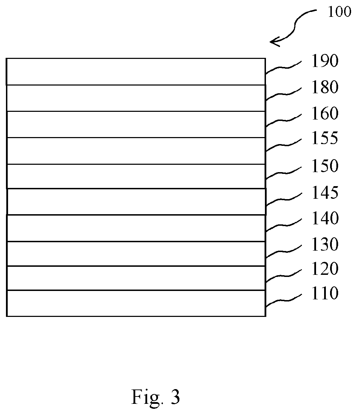

Fig. 3 is a schematic sectional view of an OLED 100, according to another exemplary embodiment of the present invention. Fig. 3 differs from Fig. 2 in that the OLED 100 of Fig. 3 comprises an electron blocking layer (EBL) 145 and a hole blocking layer (HBL) 155.

Referring to Fig. 3, the OLED 100 includes a substrate 110, an anode layer 120, a hole injection layer (HIL) 130 which may comprise a compound of formula (I), a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode layer 190.

While not shown in Fig. 1, Fig. 2 and Fig. 3, a sealing and/or capping layer may further be formed on the cathode layer 190, in order to seal the organic electronic device 100. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

### Detailed description

The invention is furthermore illustrated by the following examples which are illustrative only and non-binding.

Compounds of formula (I) may be prepared as described in EP2180029A1 and WO2016097017A1.

### Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

### General procedure for fabrication of OLEDs

For bottom emission devices, see Table 3, a 15Ω /cm² glass substrate with 90 nm ITO (available from Corning Co.) was cut to a size of 50 mm x 50 mm x 0.7 mm, ultrasonically washed with isopropyl alcohol for 5 minutes and then with pure water for 5 minutes, and washed again with UV ozone for 30 minutes, to prepare the anode layer.

Then, 88 vol.-% compound of formula F3 and 12 vol.-% compound of formula (I) according to Table 3 were co-deposited in vacuum on the anode layer, to form a HIL having a thickness of 10 nm. The composition of the HIL can be seen in Table 3.

Then, compound of formula F3 was vacuum deposited on the HIL, to form a first HTL having a thickness of 128 nm.

Then N,N-bis(4-(dibenzo[b,d]furan-4-yl)phenyl)-[1,1':4',1"-terphenyl]-4-amine (CAS 1198399-61-9) was vacuum deposited on the HTL, to form an electron blocking layer (EBL) having a thickness of 5 nm.

Then 97 vol.-% H09 (Sun Fine Chemicals, Korea) as EML host and 3 vol.-% BD200 (Sun Fine Chemicals, Korea) as fluorescent blue dopant were co-deposited on the EBL, to form a first blue-emitting EML with a thickness of 20 nm.

Then the hole blocking layer (HBL) is formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer.

Then, the electron transporting layer (ETL) having a thickness of 25 nm is formed on the hole blocking layer by co-depositing 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% LiQ.

Then, the cathode layer having a thickness of 100 nm is formed on the ETL by depositing Al at a rate of 0.01 to 1 Å/s at 10⁻⁷ mbar.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m² using an Instrument Systems CAS-140CT array spectrometer (calibrated by Deutsche Akkreditierungsstelle (DAkkS)) for each of the voltage values. The cd/A efficiency at 10 mA/cm2 is determined by interpolating the luminance-voltage and current-voltage characteristics, respectively.

In bottom emission devices, the emission is predominately Lambertian and quantified in percent external quantum efficiency (EQE). To determine the efficiency EQE in % the light output of the device is measured using a calibrated photodiode at 10 mA/cm².

In top emission devices, the emission is forward directed, non-Lambertian and also highly dependent on the mirco-cavity. Therefore, the efficiency EQE will be higher compared to bottom emission devices. To determine the efficiency EQE in % the light output of the device is measured using a calibrated photodiode at 10 mA/cm².

Lifetime LT of the device is measured at ambient conditions (20°C) and 30 mA/cm², using a Keithley 2400 sourcemeter, and recorded in hours.

The brightness of the device is measured using a calibrated photo diode. The lifetime LT is defined as the time till the brightness of the device is reduced to 97 % of its initial value.

### Technical Effect of the invention

In Table 1 and 2 are shown LUMO levels for inventive compounds A1 to A101 and comparative example 1 (=C1). As comparative compound (referred to as C1) a compound with A¹ to A³ = Phenyl and R' = CN was used. LUMO levels were calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase.

Comparative compound 1 has the formula has a LUMO level of -3.81 eV.

As can be seen in Table 1 and Table 2, the LUMO level of inventive compounds A1 to A101 is more negative compared to comparative compound C1.

A more negative LUMO level is beneficial, as matrix compounds with more negative HOMO level are enabled.

Surprisingly, it was found that the effect on lowering the LUMO may most pronounced if at least one X², X³ and/or X⁴ are selected from CR², CR³ and/or CR⁴, wherein the substituent selected from CN, partially fluorinated or perfluorinated C₁ to Cs alkyl, halogen, Cl or F, and/or at least one X², X³ and/or X⁴ are selected from N.

**Table 1: Calculated LUMO levels of compounds of formula (I) wherein A¹, A² and A³ are selected the same**

| | A¹, A² and A³ | Calculated LUMO [eV] |
|---|---|---|
| A1 | 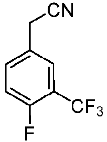 | -4.43 |
| A2 | 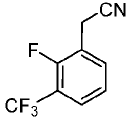 | -4.41 |
| A3 | 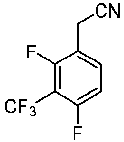 | -4.45 |
| A4 | 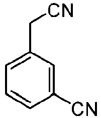 | -4.63 |
| A5 | 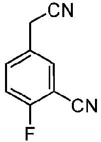 | -4.73 |
| A6 | 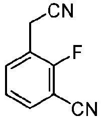 | -4.7 |
| A7 | 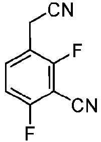 | -4.73 |
| A8 | 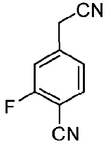 | -4.62 |
| A9 | 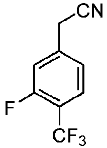 | -5.14 |
| A10 | 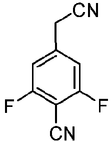 | -5.14 |
| A11 | 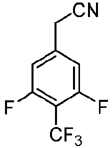 | -4.85 |
| A12 | 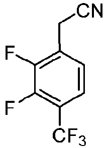 | -4.65 |
| A13 | 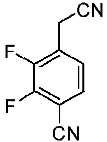 | -4.95 |
| A14 | 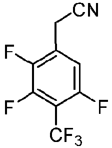 | -4.87 |
| A15 | 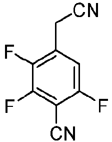 | -5.14 |
| A16 | 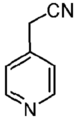 | -4.53 |
| A17 | 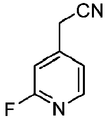 | -4.78 |
| A18 | 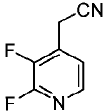 | -4.71 |
| A19 | 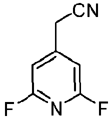 | -5.02 |
| A20 | 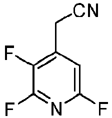 | -4.98 |
| A21 | 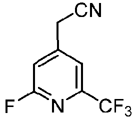 | -5.16 |
| A22 | 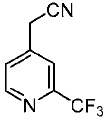 | -4.96 |
| A23 | 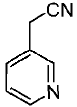 | -4.24 |
| A24 | 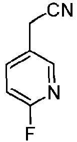 | -4.39 |
| A25 | 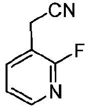 | -4.31 |
| A26 | 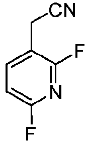 | -4.46 |
| A27 | 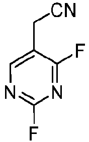 | -4.85 |
| A28 | 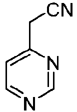 | -4.65 |
| A29 | 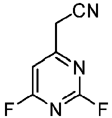 | -5.26 |
| A30 | 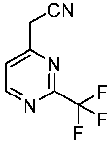 | -5.04 |
| A31 | 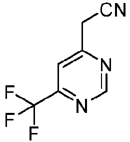 | -5.12 |
| A32 | 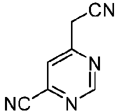 | -5.38 |

**Table 2: Calculated LUMO levels of compounds of formula (I) wherein A¹ and A² may be selected the same or differently and A¹ and A³ are not identical**

| | A¹ | A² | A³ | Calculated LUMO [eV] |
|---|---|---|---|---|
| A33 | 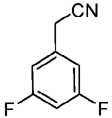 | 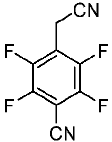 | 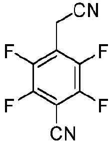 | -4.91 |
| A34 | 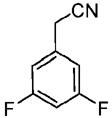 | 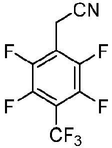 | 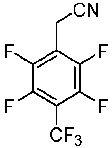 | -4.71 |
| A35 | 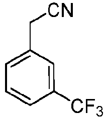 | 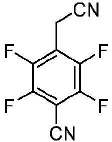 | 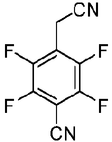 | -4.91 |
| A36 | 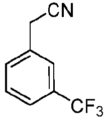 | 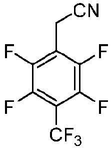 | 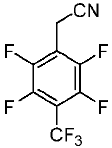 | -4.71 |
| A37 | 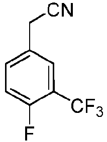 | 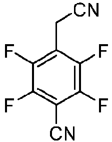 | 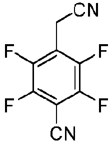 | -4.94 |
| A38 | 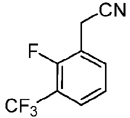 | 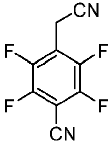 | 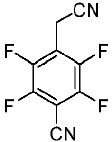 | -4.93 |
| A39 | 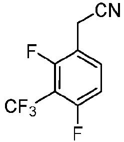 | 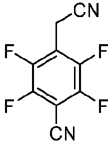 | 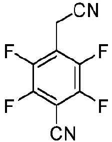 | -4.97 |
| A40 | 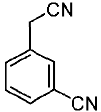 | 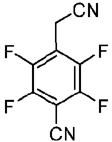 | 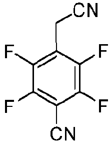 | -5 |
| A41 | 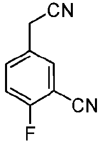 | 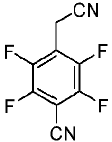 | 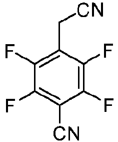 | -5.04 |
| A42 | 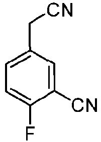 | 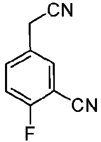 | 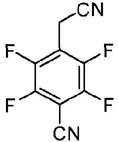 | -4.88 |
| A43 | 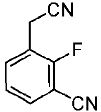 | 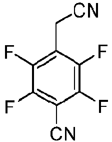 | 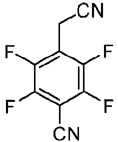 | -5.03 |
| A44 | 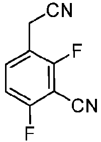 | 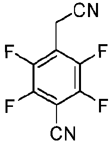 | 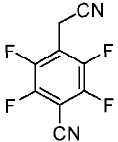 | -5.06 |
| A45 | 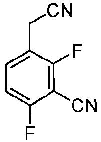 | 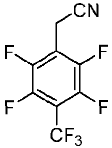 | 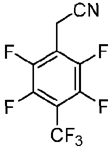 | -4.87 |
| A46 | 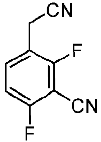 | 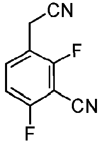 | 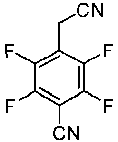 | -4.94 |
| A47 | 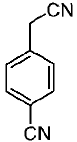 | 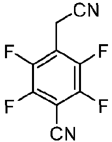 | 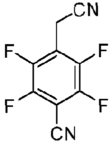 | -5.03 |
| A48 | 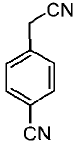 | 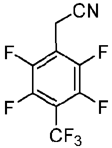 | 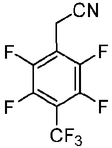 | -4.83 |
| A49 | 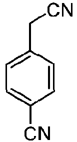 | 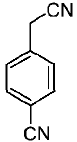 | 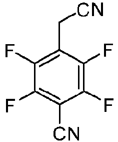 | -4.86 |
| A50 | 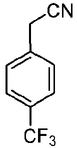 | 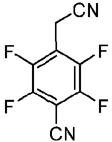 | 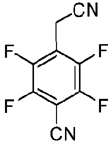 | -4.92 |
| A51 | 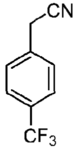 | 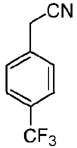 | 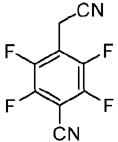 | -4.65 |
| A52 | 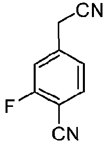 | 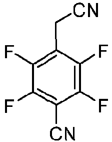 | 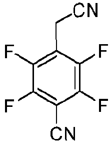 | -5.11 |
| A53 | 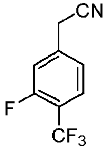 | 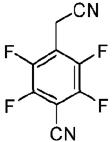 | 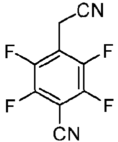 | -5 |
| A54 | 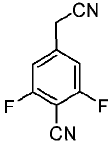 | 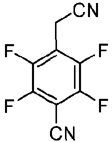 | 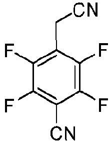 | -5.17 |
| A55 | 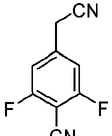 | 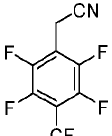 | 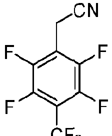 | -4.98 |
| A56 | 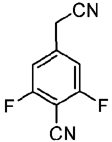 | 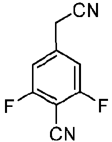 | 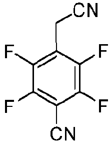 | -5.16 |
| A57 | 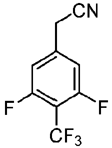 | 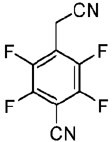 | 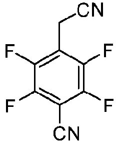 | -5.08 |
| A58 | 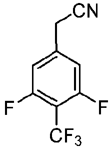 | 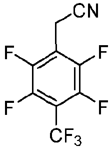 | 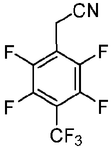 | -4.88 |
| A59 | 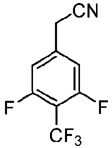 | 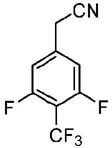 | 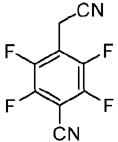 | -4.96 |
| A60 | 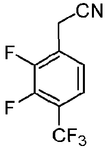 | 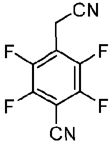 | 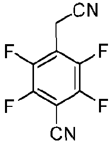 | -5.01 |
| A61 | 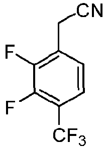 | 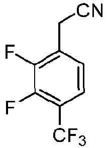 | 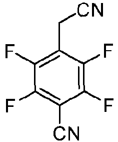 | -4.83 |
| A62 | 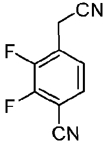 | 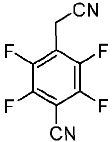 | 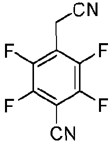 | -5.11 |
| A63 | 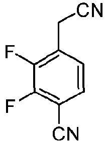 | 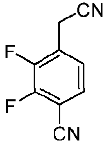 | 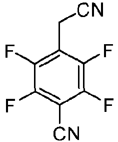 | -5.03 |
| A64 | 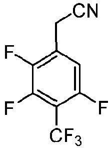 | 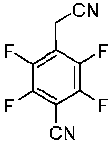 | 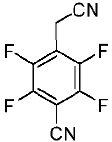 | -5.08 |
| A65 | 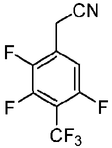 | 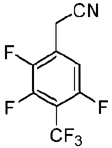 | 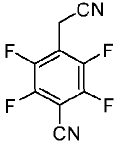 | -4.98 |
| A66 | 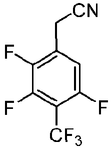 | 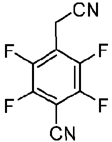 | 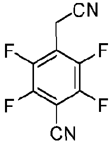 | -4.89 |
| A67 | 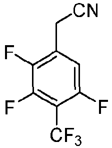 | 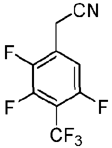 | 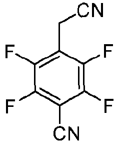 | -4.98 |
| A68 | 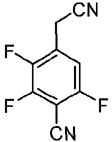 | 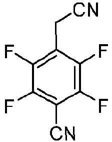 | 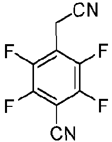 | -5.17 |
| A69 | 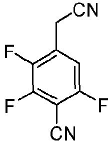 | 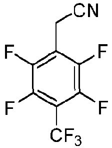 | 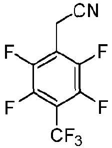 | -4.98 |
| A70 | 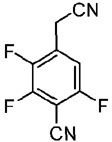 | 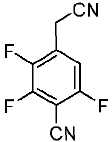 | 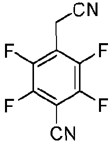 | -5.16 |
| A71 | 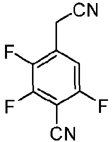 | 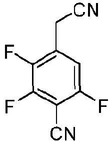 | 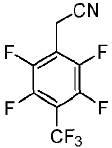 | -5.06 |
| A72 | 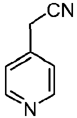 | 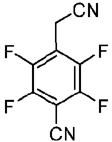 | 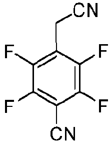 | -4.97 |
| A73 | 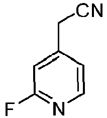 | 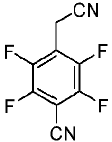 | 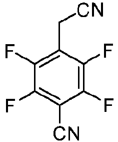 | -5.05 |
| A74 | 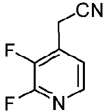 | 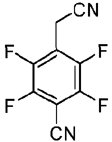 | 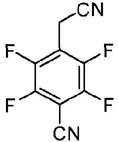 | -5.03 |
| A75 | 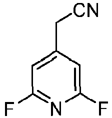 | 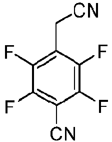 | 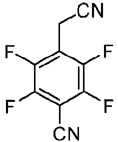 | -5.13 |
| A76 | 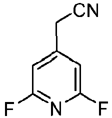 | 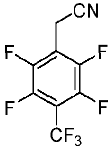 | 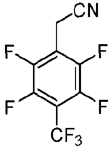 | -5.09 |
| A77 | 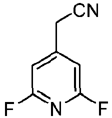 | 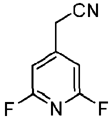 | 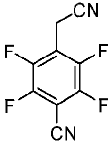 | -5.08 |
| A78 | 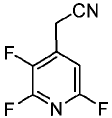 | 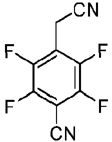 | 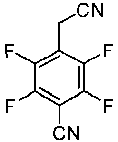 | -5.12 |
| A79 | 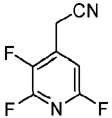 | 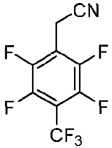 | 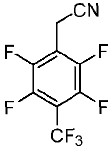 | -4.93 |
| A80 | 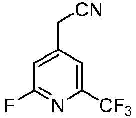 | 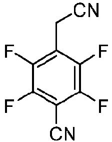 | 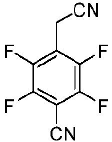 | -5.18 |
| A81 | 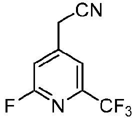 | 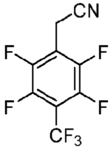 | 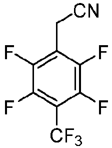 | -4.99 |
| A82 | 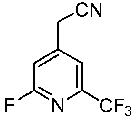 | 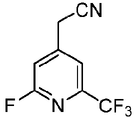 | 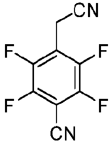 | -5.17 |
| A83 | 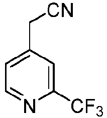 | 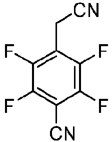 | 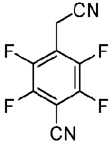 | -5.11 |
| A84 | 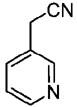 | 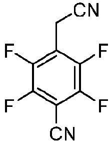 | 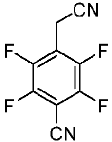 | -4.87 |
| A85 | 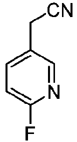 | 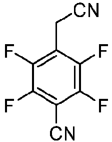 | 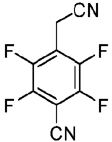 | -4.92 |
| A86 | 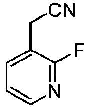 | 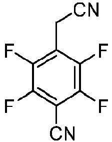 | 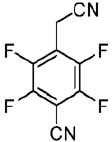 | -4.89 |
| A87 | 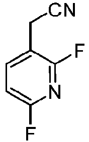 | 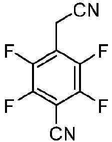 | 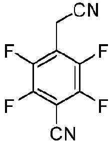 | -4.94 |
| A88 | 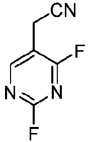 | 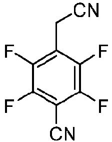 | 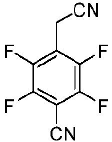 | -5.08 |
| A89 | 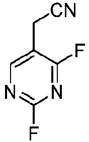 | 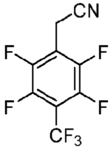 | 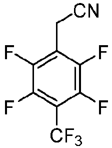 | -4.88 |
| A90 | 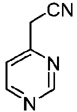 | 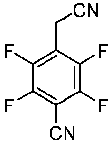 | 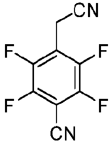 | -5.01 |
| A91 | 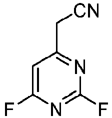 | 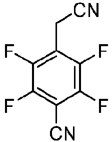 | 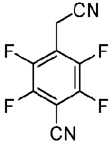 | -5.21 |
| A92 | 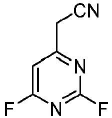 | 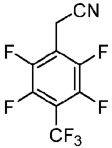 | 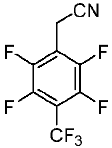 | -5.02 |
| A93 | 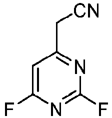 | 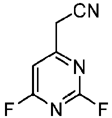 | 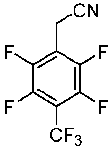 | -5.14 |
| A94 | 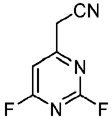 | 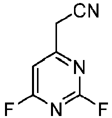 | 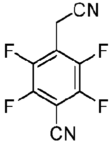 | -5.24 |
| A95 | 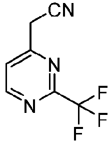 | 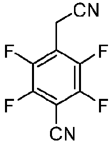 | 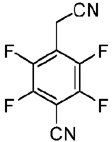 | -5.14 |
| A96 | 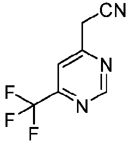 | 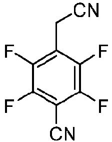 | 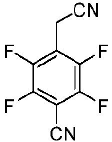 | -5.17 |
| A97 | 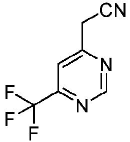 | 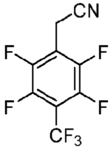 | 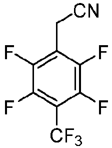 | -4.97 |
| A98 | 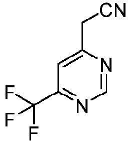 | 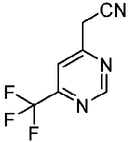 | 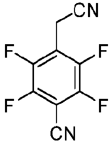 | -5.14 |
| A99 | 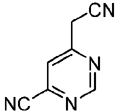 | 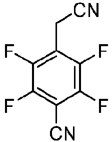 | 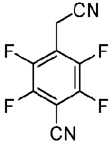 | -5.25 |
| A100 | 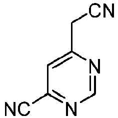 | 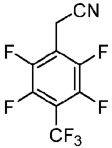 | 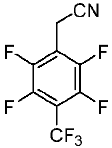 | -5.06 |
| A101 | 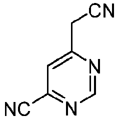 | 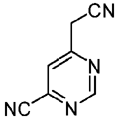 | 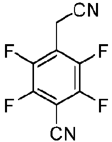 | -5.32 |

In Table 3 performance data for organic electroluminescent devices comprising a hole injection layer (HIL) comprising comparative and inventive compounds are shown:

**Table 3: Organic electronic devices comprising an hole injection layer (HIL) comprising comparative and inventive compounds.**

| | U at 10 mA/cm² [V] | EQE at 10 mA/cm² [%] | LT at 30 mA/cm² [h] |
|---|---|---|---|
| C1 | 7.5 | 7 | < 10 |
| A5 | 3.9 | 8.9 | 85 |
| A7 | 3.76 | 9.3 | 122 |
| A11 | 3.74 | 9.4 | 129 |
| A13 | 3.7 | 9.6 | 130 |
| A14 | 3.73 | 9.5 | 125 |
| A15 | 3.6 | 10.2 | 135 |
| A19 | 3.8 | 9.8 | 128 |
| A20 | 3.7 | 9.7 | 124 |
| A29 | 3.5 | 10 | 123 |
| A31 | 3.6 | 10.1 | 120 |
| A33 | 3.8 | 10.4 | 140 |
| A35 | 3.9 | 9.8 | 136 |
| A37 | 4.1 | 9.6 | 127 |
| A41 | 3.6 | 10.3 | 136 |
| A47 | 3.7 | 10.5 | 145 |
| A54 | 3.5 | 10.6 | 138 |
| A64 | 3.6 | 9.4 | 120 |
| A71 | 3.7 | 9.1 | 130 |
| A75 | 3.5 | 89 | 125 |
| A88 | 3.6 | 8.5 | 123 |
| A94 | 3.5 | 9.7 | 132 |
| A100 | 3.7 | 8 6 | 117 |

As can be seen from Table 3, the operating voltage U for devices according to invention is lower than for the comparative compounds.

As can be seen in Table 3, the external quantum efficiency EQE and lifetime LT for devices according to invention are increased compared to comparative compounds.

A low operating voltage and/or high efficiency may be beneficial for reduced power consumption and improved battery life, in particular in mobile devices.

A long lifetime is beneficial for long-time stability of devices. The particular combinations of elements and features in the above detailed embodiments are exemplary only. As those skilled in the art will recognize, variations, modifications, and other implementations of what is described herein can occur to those of ordinary skill in the art without departing from the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims. Furthermore, reference signs used in the description and claims do not limit the scope of the invention as claimed.

## Claims

1. A compound of formula (I) 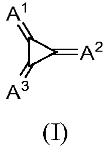 whereby A¹ is selected from formula (II) 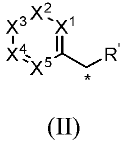
X¹ is selected from CR¹;
X² is selected from CR² or N;
X³ is selected from CR³ or N;
X⁴ is selected from CR⁴ or N;
X⁵ is selected from CR⁵ or N;
R¹ is selected from D or H,
R², R³, R⁴ and R⁵ (if present) are independently selected from CN, partially fluorinated or perfluorinated C₁ to Cs alkyl, Cl, F, D or H, whereby when any of R², R³, R⁴ and R⁵ is present, then the corresponding X², X³, X⁴ and X⁵ is not N;
with the proviso that either
- at least one R² to R⁵ is selected from selected from CN, partially fluorinated or perfluorinated C₁ to Cs alkyl, Cl, F; or
- at least one X² to X⁵ is N;
A² and A³ are independently selected from formula (III) 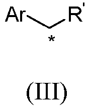
wherein Ar is independently selected from substituted or unsubstituted C₆ to C₁₈ aryl and substituted or unsubstituted C₂ to C₁₈ heteroaryl, wherein the substituents on Ar are independently selected from CN, partially or perfluorinated C₁ to C₆ alkyl, Cl, F, D; and R' is selected from Ar, substituted or unsubstituted C₆ to C₁₈ aryl or C₃ to C₁₈ heteroaryl, partially flurorinated or perfluorinated C₁ to C₈ alkyl, F or CN;and wherein the asterix "*" denotes the binding position;
wherein the LUMO level of compound of formula (I) when calculated with the program package TURBOMOLE V6.5 (TURBOMOLE GmbH, Litzenhardtstrasse 19, 76135 Karlsruhe, Germany) by applying the hybrid functional B3LYP with a 6-31G* basis set in the gas phase is selected in the range of ≤ -4.7 eV and ≥ -5.25 eV ;
wherein the following compounds are excluded:

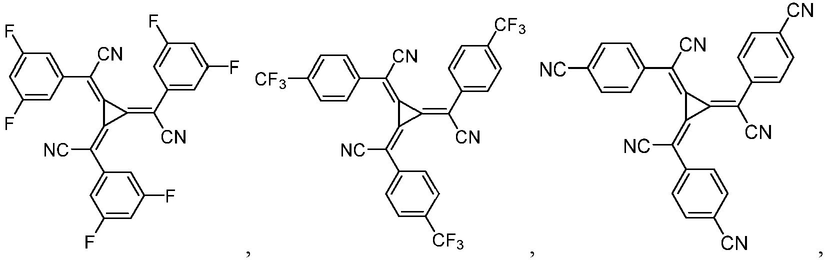

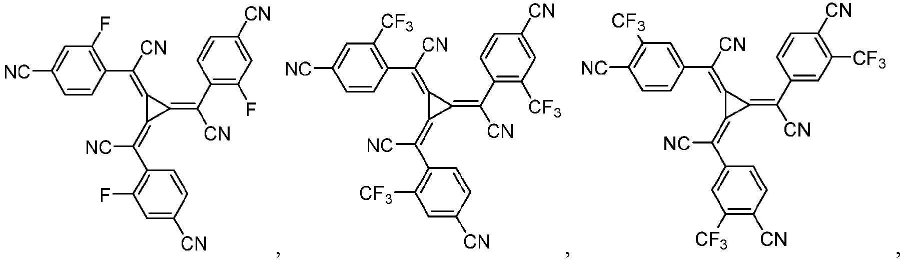

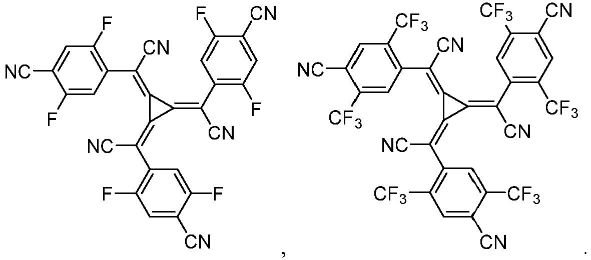

2. The compound of Claim 1, selected of the formula (IV) 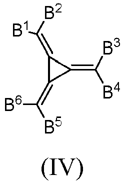 whereby B¹ is selected from formula (V) 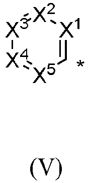 B³ and B⁵ are Ar and B², B⁴ and B⁶ are R'.

3. The compound of Claim 1 or 2 whereby formula (II) does not include the following moieties:

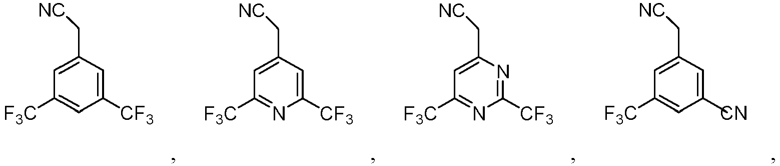

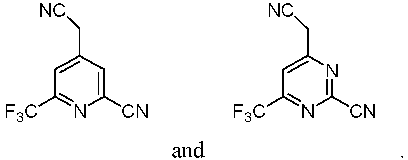

4. The compound of any of the Claims 1 to 3 whereby formula (III) does not include the following moieties: 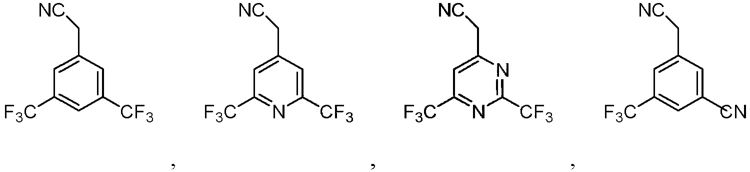 and

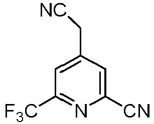

5. The compound of any of the claims 1 to 4, whereby A¹ differs from A² and/or A³.

6. The compound of any of the claims 1 to 5, whereby X³ is selected from N or CR³, wherein R³ is selected from CN, partially flurorinated or perfluorinated C₁ to Cs alkyl.

7. The compound of any of the claims 1 to 6, whereby one of R² and R³ is selected from CN, partially flurorinated or perfluorinated C₁ to Cs alkyl and at least one R² to R⁵ is independently selected from CN, partially flurorinated or perfluorinated C₁ to Cs alkyl, Cl, F.

8. The compound of any of the Claims 1 to 7, whereby R⁵ is D or H.

9. The compound any of the claims 1 to 8, whereby at least two out of R² to R⁵ are independently selected from Cl, F.

10. An organic electronic device (100) comprising an anode player (120), a cathode layer (190) and at least one organic semiconductor layer, wherein the at least one organic semiconductor layer is arranged between the anode layer and the cathode layer; and wherein the at least one organic semiconductor layer comprises a compound of any of the claims 1 to 9.

11. The organic electronic device of claim 10, whereby the organic semiconductor layer comprises a composition comprising a compound of formula (IV) and at least one compound of formula (IVa) to (IVd)

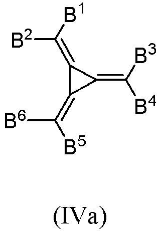

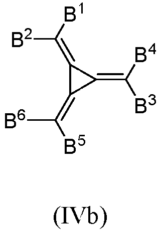

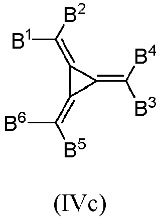

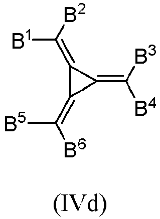

12. The organic electronic device of claim 10 or 11, whereby the organic electronic device comprises at least one photoactive layer and the at least one of the at least one organic semiconductor layers is arranged between the anode layer and the at least one photoactive layer.

13. The organic electronic device of any of the claims 10 to 12, whereby the organic electronic device comprises at least two photoactive layers, wherein at least one of the at least one organic semiconductor layers is arranged between the first and the second photoactive layer.

14. The organic electronic device of any of the claims 10 to 13, whereby the electronic organic device is an electroluminescent device, preferably an organic light emitting diode.

15. A display device comprising an organic electronic device according to any of the claims 10 to 14.

## Patentansprüche

1. Verbindung der Formel (I) 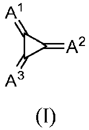 wobei A¹ ausgewählt ist aus Formel (II) 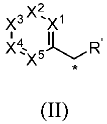
X¹ aus CR¹ ausgewählt ist;
X² aus CR² oder N ausgewählt ist;
X³ aus CR³ oder N ausgewählt ist;
X⁴ aus CR⁴ oder N ausgewählt ist;
X⁵ aus CR⁵ oder N ausgewählt ist;
R¹ aus D oder H ausgewählt ist,
R², R³, R⁴ und R⁵ (falls vorhanden) unabhängig aus CN, teilfluoriertem oder perfluoriertem C₁- bis C₈-Alkyl, Cl, F, D oder H ausgewählt sind, wobei dann, wenn eines von R², R³, R⁴ und R⁵ vorliegt, das entsprechende X², X³, X⁴ und X⁵ nicht für N steht;
mit der Maßgabe, dass entweder
- mindestens ein R² bis R⁵ aus CN, teilfluoriertem oder perfluoriertem C₁- bis C₈-Alkyl, Cl, F ausgewählt ist;
oder
- mindestens ein X² bis X⁵ für N steht;
A² und A³ unabhängig ausgewählt sind aus Formel (III) 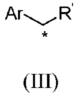 wobei Ar unabhängig aus substituiertem oder unsubstituiertem C₆- bis C₁₈-Aryl und substituiertem oder unsubstituiertem C₂- bis C₁₈-Heteroaryl ausgewählt ist, wobei die Substituenten an Ar unabhängig aus CN, teil- oder perfluoriertem C₁- bis C₆-Alkyl, Cl, F, D ausgewählt sind; und
R' aus Ar, substituiertem oder unsubstituiertem C₆- bis C₁₈-Aryl oder C₃- bis C₁₈-Heteroaryl, teilfluoriertem oder perfluoriertem C₁- bis C₈-Alkyl, F oder CN ausgewählt ist; und
wobei das Sternchen "*" die Bindungsposition bezeichnet; wobei das LUMO-Niveau der Verbindung der Formel (I) bei Berechnung mit dem Programmpaket TURBOMOLE V6.5 (TURBOMOLE GmbH, Litzenhardtstraße 19, 76135 Karlsruhe, Deutschland) durch Anwenden des Hybridfunktionals B3LYP mit einem 6-31G*-Basissatz in der Gasphase im Bereich von ≤ -4,7 eV und ≥ -5,25 eV ausgewählt ist;
wobei die folgenden Verbindungen ausgeschlossen sind:

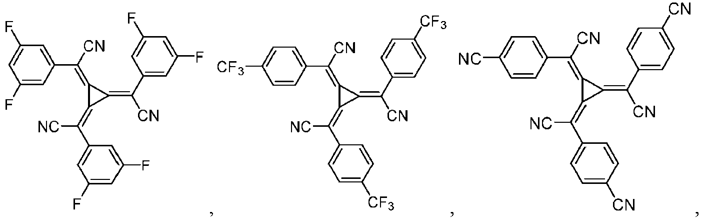

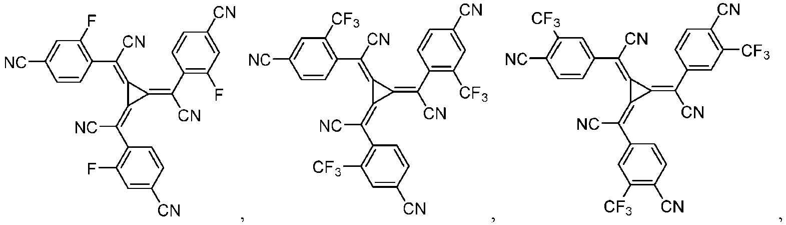

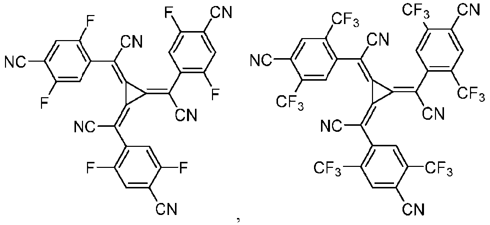

2. Verbindung nach Anspruch 1, ausgewählt aus der Formel (IV) 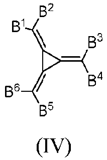 wobei B¹ aus Formel (V) 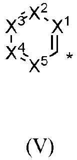 ausgewählt ist, B³ und B⁵ für Ar stehen und B², B⁴ und B⁶ für R' stehen.

3. Verbindung nach Anspruch 1 oder 2, wobei Formel (II) die folgenden Gruppierungen nicht enthält:

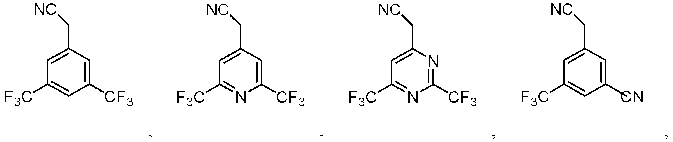

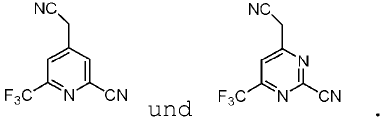

4. Verbindung nach einem der Ansprüche 1 bis 3, wobei Formel (III) die folgenden Gruppierungen nicht enthält: 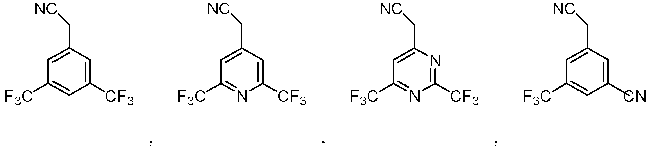 und

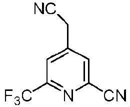

5. Verbindung nach einem der Ansprüche 1 bis 4, wobei A¹ von A² und/oder A³ verschieden ist.

6. Verbindung nach einem der Ansprüche 1 bis 5, wobei X³ aus N oder CR³ ausgewählt ist, wobei R³ aus CN, teilfluoriertem oder perfluoriertem C₁- bis C₈-Alkyl ausgewählt ist.

7. Verbindung nach einem der Ansprüche 1 bis 6, wobei eines von R² und R³ aus CN, teilfluoriertem oder perfluoriertem C₁- bis C₈-Alkyl ausgewählt ist und mindestens ein R² bis R⁵ unabhängig aus CN, teilfluoriertem oder perfluoriertem C₁- bis C₈-Alkyl, Cl, F ausgewählt ist.

8. Verbindung nach einem der Ansprüche 1 bis 7, wobei R⁵ für D oder H steht.

9. Verbindung nach einem der Ansprüche 1 bis 8, wobei mindestens zwei von R² bis R⁵ unabhängig aus Cl, F ausgewählt sind.

10. Organische elektronische Vorrichtung (100), umfassend eine Anodenschicht (120), eine Kathodenschicht (190) und mindestens eine organische Halbleiterschicht, wobei die mindestens eine organische Halbleiterschicht zwischen der Anodenschicht und der Kathodenschicht angeordnet ist und wobei die mindestens eine organische Halbleiterschicht eine Verbindung nach einem der Ansprüche 1 bis 9 umfasst.

11. Organische elektronische Vorrichtung nach Anspruch 10, wobei die organische Halbleiterschicht eine Zusammensetzung umfasst, die eine Verbindung der Formel (IV) und mindestens eine Verbindung der Formel (IVa) bis (IVd) umfasst.

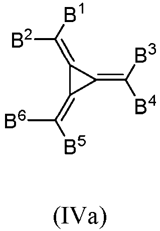

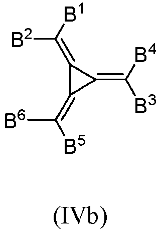

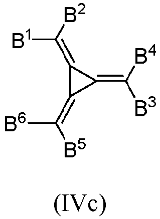

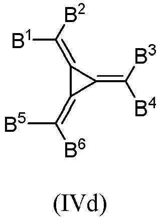

12. Organische elektronische Vorrichtung nach Anspruch 10 oder 11, wobei die organische elektronische Vorrichtung mindestens eine photoaktive Schicht umfasst und die mindestens eine der mindestens einen organischen Halbleiterschichten zwischen der Anodenschicht und der mindestens einen photoaktiven Schicht angeordnet ist.

13. Organische elektronische Vorrichtung nach einem der Ansprüche 10 bis 12, wobei die organische elektronische Vorrichtung mindestens zwei photoaktive Schichten umfasst, wobei mindestens eine der mindestens einen organischen Halbleiterschichten zwischen der ersten und der zweiten photoaktiven Schicht angeordnet ist.

14. Organische elektronische Vorrichtung nach einem der Ansprüche 10 bis 13, wobei es sich bei der elektronischen organischen Vorrichtung um eine Elektrolumineszenzvorrichtung, vorzugsweise eine organische Leuchtdiode, handelt.

15. Anzeigevorrichtung, umfassend eine organische elektronische Vorrichtung nach einem der Ansprüche 10 bis 14.

## Revendications

1. Composé de formule (I) 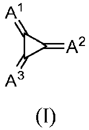 A¹ étant choisi parmi la formule (II) 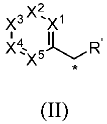
X¹ étant choisi parmi CR¹ ;
X² étant choisi parmi CR² ou N ;
X³ étant choisi parmi CR³ ou N ;
X⁴ étant choisi parmi CR⁴ ou N ;
X⁵ étant choisi parmi CR⁵ ou N ;
R¹ étant choisi parmi D ou H,
R², R³, R⁴ et R⁵ (le cas échéant) étant choisis indépendamment parmi CN, alkyle en C₁ à C₈ partiellement fluoré ou perfluoré, Cl, F, D ou H, où lorsque l'un quelconque parmi R², R³, R⁴, et R⁵ est présent, alors les X², X³, X⁴, et X⁵ correspondants ne sont pas N ;
étant entendu que soit
- au moins un R² à R⁵ est choisi parmi CN, alkyle en C₁ à C₈ partiellement fluoré ou perfluoré, Cl, F ; ou
- au moins un X² à X⁵ est N ;
A² et A³ étant indépendamment choisis parmi la formule (III) 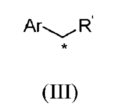
Ar étant indépendamment choisi parmi aryle en C₆ à C₁₈ substitué ou non substitué et hétéroaryle en C₂ à C₁₈ substitué ou non substitué, les substituants sur Ar étant indépendamment choisis parmi CN, alkyle en C₁ à C₆ partiellement fluoré ou perfluoré, Cl, F, D ; et
R' étant choisi parmi Ar, aryle en C₆ à C₁₈ ou hétéroaryle en C₃ à C₁₈ substitué ou non substitué, alkyle en C₁ à C₈ partiellement fluoré ou perfluoré, F ou CN ; et l'astérisque « * » désignant la position de liaison ;
le niveau de LUMO du composé de formule (I), lorsqu'il est calculé avec le progiciel TURBOMOLE V6.5 (TURBOMOLE GmbH, Litzenhardtstrasse 19, 76135 Karlsruhe, Allemagne) par application du B3LYP fonctionnel hybride avec un ensemble de base 6-31G* définie dans la phase gazeuse étant choisi dans la plage de ≤ -4,7 eV et ≥ -5,25 eV ; les composés suivants étant exclus :

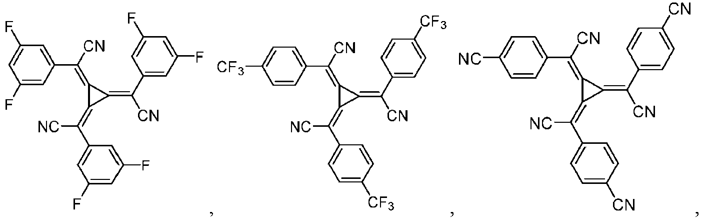

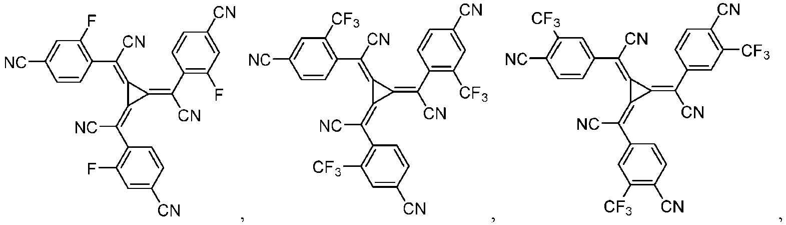

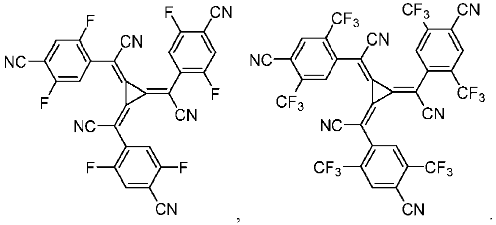

2. Composé selon la revendication 1, choisi parmi la formule (IV) 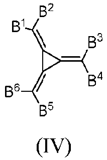 B¹ étant choisi parmi la formule (V) 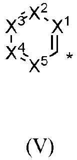 B³ et B⁵ étant Ar et B², B⁴ et B⁶ étant R' .

3. Composé selon la revendication 1 ou 2, dans lequel la formule (II) ne comporte pas les groupements suivants :

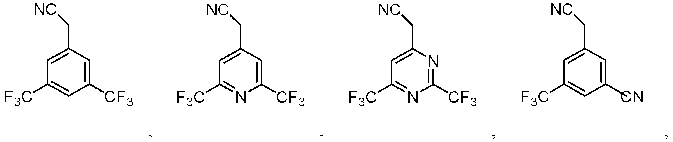

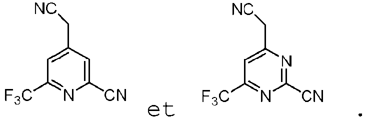

4. Composé selon l'une quelconque des revendications 1 à 3, dans lequel la formule (III) ne comporte pas les groupements suivants : 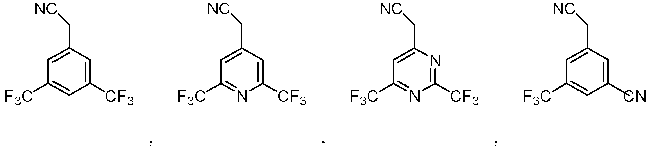 et

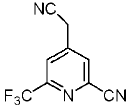

5. Composé selon l'une quelconque des revendications 1 à 4, dans lequel A¹ diffère de A² et/ou A³.

6. Composé selon l'une quelconque des revendications 1 à 5, dans lequel X³ est choisi parmi N ou CR³, où R³ est choisi parmi CN, alkyle en C₁ à C₈ partiellement fluoré ou perfluoré.

7. Composé selon l'une quelconque des revendications 1 à 6, dans lequel au moins l'un parmi R² et R³ est choisi parmi CN, alkyle en C₁ à C₈ partiellement fluoré ou perfluoré, et au moins l'un parmi R² à R⁵ est indépendamment choisi parmi CN, alkyle en C₁ à C₈ partiellement fluoré ou perfluoré, Cl, F.

8. Composé selon l'une quelconque des revendications 1 à 7, dans lequel R⁵ est D ou H.

9. Composé selon l'une quelconque des revendications 1 à 8, dans lequel au moins deux parmi R² à R⁵ sont indépendamment choisis parmi Cl, F.

10. Dispositif électronique organique (100) comprenant une couche d'anode (120), une couche de cathode (190) et au moins une couche de semi-conducteur organique, l'au moins une couche de semi-conducteur organique étant disposée entre la couche d'anode et la couche de cathode, et l'au moins une couche de semi-conducteur organique comprenant un composé selon l'une quelconque des revendications 1 à 9.

11. Dispositif électronique organique selon la revendication 10, dans lequel la couche de semi-conducteur organique comprend une composition comprenant un composé de formule (IV) et au moins un composé de formule (IVa) à (IVd)

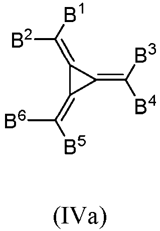

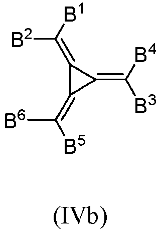

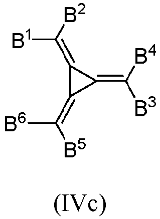

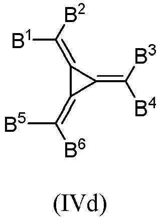

12. Dispositif électronique organique selon la revendication 10 ou 11, dans lequel le dispositif électronique organique comprend au moins une couche photoactive et l'au moins une parmi l'au moins une couche de semi-conducteur organique est disposée entre la couche d'anode et l'au moins une couche photoactive.

13. Dispositif électronique organique selon l'une quelconque des revendications 10 à 12, dans lequel le dispositif électronique organique comprend au moins deux couches photoactives, au moins une des au moins une couche de semi-conducteur organique étant disposée entre la première et la seconde couche photoactive.

14. Dispositif électronique organique selon l'une quelconque des revendications 10 à 13, dans lequel le dispositif électronique organique est un dispositif électroluminescent, de préférence une diode électroluminescente organique.

15. Dispositif d'affichage comprenant un dispositif électronique organique selon l'une quelconque des revendications 10 à 14.
